# EUROPÄISCHE PATENTANMELDUNG

(11) **EP 4 553 500 A1**
(43) Veröffentlichungstag der Anmeldung: **14.05.2025**
(21) Anmeldenummer: 23209142.1
(22) Anmeldetag: 10.11.2023
(51) Int. Cl.: G01N 33/543, G01N 21/64, G01N 33/58

(54) **VERFAHREN ZUM NACHWEIS UND ZUR BESTIMMUNG DER BINDUNGSSTÄRKE WENIGSTENS EINES ANALYTEN IN EINER PROBE**

(71) Anmelder: Attomol GmbH Molekulare Diagnostika, 03205 Bronkow (DE)
(72) Erfinder: Lehmann, Werner, 03205 Bronkow (DE); Berger, Enrico, 01968 Senftenberg Niemtsch (DE); Sädler, Eric, 01968 Senftenberg (DE)
(74) Vertreter: Gulde & Partner

(57) **Zusammenfassung**

Die vorliegende Erfindung betrifft ein Verfahren zum Nachweis und zur Bestimmung der Bindungsstärke wenigstens eines Analyten in einer Probe, das auf dem Prinzip eines Immunoassays basiert. Das Verfahren ermöglicht es, einen Nachweis und nachfolgend die Charakterisierung der Bindungseigenschaften von Analyten in einem Test durchzuführen. Ferner wird ein Kit zur Durchführung des erfindungsgemäßen Verfahrens bereitgestellt.

## Beschreibung

### Gebiet der Erfindung

Die vorliegende Erfindung betrifft ein Verfahren zum Nachweis und zur Bestimmung der Bindungsstärke wenigstens eines Analyten in einer Probe, das auf dem Prinzip eines Immunoassays basiert. Das Verfahren ermöglicht es einen Nachweis und nachfolgend die Charakterisierung der Bindungseigenschaften von Analyten in einem Test durchzuführen. Ferner wird ein Kit zur Durchführung des erfindungsgemäßen Verfahrens bereitgestellt.

### Hintergrund der Erfindung

Neben dem Nachweis eines klinisch relevanten Analyten in einer Probe, spielt ebenso die Bindungsstärke des Analyten an sein Target eine Rolle. Die Affinität, mit der der Analyt beispielsweise an seine Rezeptoren bindet oder mit der er von abbauenden Enzymen gebunden wird, kann Aussagen über seine pathophysiologische Bedeutung im Organismus ermöglichen. Von der Affinität kann somit abhängen, wie wirksam ein Arzneimittel ist und wie schnell es vom Patienten metabolisiert werden kann. Von besonderem Interesse ist die Bindungsstärke von Antikörpern, die durch die Applikation von Vakzinen induziert werden, da diese mit einer hohen Bindungsstärke therapeutisch am relevantesten sind. Eine starke Bindung der gebildeten Antikörper an ihr spezifisches Antigen, wie beispielsweise ein Hüllprotein eines Virus, kann effektiv zur Neutralisierung des Virus beitragen.

Auch bei Antikörpern, die ohne Vakzinierung in Folge einer Infektionserkrankung gebildet werden, kann die Bindungsstärke der Antikörper darüber entscheiden, ob ein effektiver Schutz des Patienten bei erneutem Erregerkontakt gegeben ist. Ein weiteres Beispiel sind Autoantikörper, die als Quelle vielfältiger Autoimmunerkrankungen eine Rolle spielen. Vor dem Hintergrund, dass viele Menschen, die noch nicht erkrankt sind, bereits Autoantikörper bilden und daher ein größeres Risiko besitzen zu erkranken, werden die Autoantikörper bestimmt, um entsprechende Risikopatienten zu identifizieren. Daher ist neben dem Nachweis des Vorhandenseins von Autoantikörpern, auch die Bindungsstärke dieser Autoantikörper Gegenstand der klinischen Forschung der letzten zehn Jahre. Es konnte gezeigt werden, dass in Abhängigkeit des spezifischen Autoantikörpers und des betrachteten klinischen Bildes niedrigaffine Antikörper oder hochaffine Antikörper für eine Autoimmunerkrankung wegweisend sein können.

Die Bindungsstärke eines Antikörpers an seine Antigendeterminante, dem Epitop, bezeichnet man als Antikörperaffinität, sie ist unter anderem abhängig von pH-Wert und lonenstärke. Die Summe der Bindungsstärken einer Gruppe polyklonaler Antikörper, beispielsweise in einem Serum, an ein Antigen, wird mit dem Begriff Antikörperavidität ausgedrückt.

Das Immunsystem reagiert auf eine Infektion zunächst mit der Bildung niedrig-avider Antikörper gegen die Antigene der Erreger. Mit fortschreitender Krankheitsdauer passt der Organismus das spezifische Antikörper Immunglobulin-G (IgG) den Antigenen immer genauer an, so dass die Avidität zunimmt. Ist in einem Serum bereits hoch-avides IgG nachweisbar, dann lässt dies den Schluss zu, dass eine Infektion schon länger zurückliegen muss.

Im Stand der Technik sind zahlreiche Methoden zum Nachweis eines Analyten in einer Probe bekannt. Die WO 2009/055382 A2 offenbart beispielsweise Immunoassays zum Nachweis oder zur Quantifizierung mindestens eines Analyten in einer Testprobe, die eine verbesserte Empfindlichkeit oder Empfindlichkeit und Spezifität aufweisen. In der WO 2013/096851 A1 wird der Nachweis und/oder die Quantifizierung von Analyten in einer Probe durch Multiplex-Systeme beschrieben. In der WO 2013/144615 A1 wird ein biologisches Reagenz bereitgestellt, wobei das Reagenz eine Mischung von Analyten umfasst, die im Wesentlichen in den gleichen relativen Konzentrationen wie bei der Probenahme vorliegen, wobei die Mischung von Analyten mindestens einen Zielanalyten umfasst, der mit einem nachweisbaren Analyt-bindenden Mittel markiert ist. Das Reagenz findet in diagnostischen Tests Verwendung, um das Vorhandensein oder die Konzentration mindestens eines Zielanalyten in einer Testprobe zu bestimmen. In der WO 99/54736 A1 werden ein Bindungsassay im Mikromaßstab, ein Analytbindungsarray und Kits offenbart, die das Massenwirkungsgesetz nutzen, um Analyten aus einer flüssigen Probe zu gewinnen. Dies wird durch die Herstellung von Sorbenszonen erreicht, die eine bis zu zehnfache Bindungskapazität pro Flächeneinheit aufweisen, die im Allgemeinen auf Polystyrol-Mikrotiterplatten erreicht wird. Dieser Ansatz, gepaart mit der direkten Fluoreszenzdetektion im Nahinfrarot (NIR), führt zu maximaler Signalintensität und niedrigem Hintergrund für optimale Empfindlichkeit. WO 2018/077783 A1 offenbart ein Verfahren zum Nachweis eines Analyten, umfassend a) das Kontaktieren des Analyten, eines Bindemittels und einer festen Oberfläche, um einen an eine feste Oberfläche gebundene Bindungskomplex zu bilden, b) das Spalten mindestens einer kovalenten Bindung innerhalb des Analyten und/oder innerhalb des Bindungsmittels, das in dem Bindungskomplex enthalten ist, und dadurch Freisetzung des Analyten oder eines Fragments davon aus dem Bindungskomplex, und c) Nachweis mindestens eines Fragmentes des Analyten und dadurch Nachweis des Analyten.

Für die Bestimmung der Avidität werden die aus dem Stand der Technik bekannten Enzymelinked Immunosorbentassay (ELISA)-Systeme, Immunblot- und Immunfluoreszenz-Techniken eingesetzt. Dabei wird beispielsweise zwischen dem ersten Inkubationsschritt (Patientenserum) und dem zweiten (markierter Antikörper) ein Verdrängungsschritt geschaltet. Eine Inkubation erfolgt in der Regel mit einer Harnstofflösung oder mit anderen chaotropen Reagenzien, die in der Lage sind, niedrig-avide Antikörper von ihrem Antigen abzusprengen, wobei die hoch-aviden Antikörper fest mit dem Antigen verbunden bleiben. Das Resultat wird zu dem eines Parallelansatzes ohne Verdrängungsschritt ins Verhältnis gesetzt und der relative Aviditätsindex (RAI) berechnet. Dabei wird das Ergebnis der Inkubation mit Harnstoff dividiert durch das Ergebnis der Inkubation ohne Harnstoff herangezogen. Bei einem RAI unter 40 % im ELISA-Test, liegen niedrig-avide Antikörper vor, was als Indiz auf eine frische Infektion hindeuten kann. Im Falle der indirekten Immunfluoreszenz, werden Differenzen von zwei oder mehr quadratischen Titerstufen als Hinweis auf das Vorliegen niedrig-avider Antikörper betrachtet.

Ein weiteres Verfahren, welches für die Bestimmung der Avidität genutzt werden kann, ist die Surface Plasmon Resonance. Mit diesem Verfahren ist es möglich Bindungskinetiken von Antikörpern an das jeweilige Antigen zu bestimmen, um z.B. die Affinitäten von monoklonalen Antikörpern in Form von Bindungskonstanten zu beschreiben. Dazu ist keine zusätzliche Markierung des Analyten, hier des Antikörpers, nötig, da markierungsfrei gemessen werden kann. Die markierungsfreie Messung verhindert vor allem bei kleineren Analytmolekülen, dass die molekularen Bindungseigenschaften des Analyten verfälscht werden. Um die Bindungskonstanten zu ermitteln, müssen allerdings Messungen mit verschiedenen Konzentrationen des Analyten vorgenommen werden.

Bei einem weiteren Verfahren zur Bestimmung der Affinität von molekularen Bindungspartnern, dem Thermal Shift Assay, wird eine Temperaturerhöhung der Reaktionslösung genutzt, um die Interaktion von Bindungspartnern aufzulösen. Mindestens einer der Bindungspartner trägt eine Fluoreszenzmarkierung, deren Fluoreszenzeigenschaften sich bei der Bindung an den Bindungspartner verändern. Als Fluoreszenzmarkierung stehen eine Reihe von Fluoreszenzfarbstoffen zur Verfügung, die an einen der Bindungspartner chemisch gekoppelt werden müssen, die so zur Veränderung der Bindungspartner führen können. Die verwendeten Fluoreszenzfarbstoffe müssen im Temperaturgradienten ein messbares Verhalten aufweisen, welches sich im gebundenen Zustand des Analyten vom ungebundenen Zustand unterscheidet. Auch dieses Verfahren kann, wie die Surface Plasmon Resonance, im homogenen Testformat angewendet werden, was einerseits Arbeitsschritte spart, andererseits aber gewährleisten muss, dass die Probenmatrix den Messvorgang nicht beeinflusst.

Nachteilig an den im Stand der Technik beschriebenen Verfahren ist jedoch, dass gerade komplexe Matrices, wie z.B. Serum, den Messvorgang negativ beeinflussen können und daher die Ergebnisse oft nicht reproduzierbar sind. In Folge können die Ergebnisse nicht zuverlässig ausgewertet werden oder die Sensitivität der Messung kann verfälscht werden, ohne dass dieser Fehler erkannt wird. Ebenfalls kann es durch Bindung von Matrixmolekülen an die Fluoreszenzmarkierung der Analyten zu einer Verfälschung der Spezifität des Nachweises kommen. Darüber hinaus müssen bei allen im Stand der Technik beschriebenen Verfahren zum Antikörpernachweis zusätzlich Untersuchungen zur Bestimmung der Avidität durchgeführt werden. Dies geht mit zusätzlichem Kosten- und Zeitaufwand einher, was einer Etablierung in der Routineanalytik entgegensteht.

Ein weiterer wesentlicher Nachteil des Standes der Technik besteht darin, dass die Avidität der Antikörper einer Probe summativ erfasst wird, da durch die Messtechnik einzelne Moleküle nicht unterscheidbar erfasst werden können. Die humorale Immunantwort ist jedoch meist ein polyklonales Geschehen, d.h. dass die Antikörper von mehreren B-Zell-Klonen gebildet werden, deren Antikörper sich in Sensitivität und Spezifität unterscheiden. Die Sensitivität und Spezifität der Probe bilden somit ein Mittel der Sensitivitäten und Spezifitäten aller Antikörperklone. Dadurch ist es zum Beispiel möglich, dass wenige hochaffine Antikörper mit Krankheitsrelevanz in einer großen Menge niederaffiner Antikörper nicht erkannt werden können. Gerade zu Beginn einer humoralen Immunantwort überwiegen niedrigaffine Klone, die durch B-Zellreifung an Affinität und damit an Bedeutung als Auslöser einer Autoimmunerkrankung gewinnen können.

Aufgabe der vorliegenden Erfindung ist es, einen oder mehrere Nachteile des Standes der Technik zu vermindern oder zu vermeiden. Insbesondere ist es eine Aufgabe der vorliegenden Erfindung, ein Verfahren zum gleichzeitigen Nachweis und zur Bestimmung der Bindungsstärke wenigstens eines Analyten summativ und bei Bedarf zur Bestimmung von einzelnen Molekülen bereitzustellen. Ebenso ist es eine Aufgabe, ein Verfahren bereitzustellen, das auf einfache Weise automatisiert und auch in bestehende automatisierte Systeme, wie beispielsweise solche, die Nukleinsäuren aufreinigen können, integriert werden kann.

### Zusammenfassung der Erfindung

Die vorliegende Erfindung ist definiert durch die anhängenden Ansprüche.

Die vorliegende Erfindung stellt ein Verfahren zum sensitiven summativen und/oder Einzelmolekülnachweis eines Analyten und seiner Avidität dar, wobei in einem Test die Analyten zunächst an ein Targetmolekül gebunden werden und der Nachweis des Analyten erfolgt und nachfolgend die Bindung des Analyten dissoziiert wird, um dessen Avidität zu bestimmen.

Das Verfahren hat den Vorteil, dass der summative und/oder Einzelmolekülnachweis des Analyten und die nachfolgende Charakterisierung der Bindung des Analyten in einem Test erfolgen kann. Dabei wird für die Charakterisierung der Bindung der Analyten der Umstand genutzt, dass der Analyt nach dem Nachweis bereits gebunden an der festen Phase, und durch die Nachweisfluoreszenzmarkierung fluoreszenzmarkiert im Reaktionsraum vorliegt. Es wird somit nur noch ein Test benötigt, da die Bestimmung der Avidität auf Tests mit einem positiven Nachweis des Analyten beschränkt werden können, was die Analysezeit erheblich reduziert und Material einspart. Darüber hinaus kann durch die feine Graduierung der Dissoziation der Bindung von Target- und Analytmolekülen die Aufzeichnung von Dissoziationskinetiken ermöglicht werden.

Ebenfalls vorteilhaft ist es, dass Testsysteme wie beispielsweise Beadassays, ohne weitere Modifizierung für den Nachweis eines Analyten und zur Bestimmung seiner Bindungsstärke eingesetzt werden können.

Ein weiterer Vorteil des Verfahrens besteht darin, dass über die Wahl der Nachweisfluoreszenzmarkierung die Möglichkeit besteht, die Analyten summativ oder als Einzelmoleküle zu detektieren und die Affinität zu untersuchen.

Insgesamt ermöglicht das erfindungsgemäße Verfahren auf einfache Weise den Vergleich der Bindung von Analyten an nichtmodifizierte und modifizierte Targetmoleküle.

Das erfindungsgemäße Verfahren weist hinsichtlich der Anforderungen für die Prozessierung Ähnlichkeiten mit einer Nukleinsäurepräparation bzw. der Abarbeitung von Immunoassays auf und kann aus diesem Grund auf einfache Weise in bestehende automatisierte Systeme, die für die Isolierung und Nachweis von Nukleinsäuren und die Abarbeitung von Immunoassays ausgerichtet sind, integriert werden. Dadurch wird ferner auch ein automatisiertes System bereitgestellt, das es ermöglicht, sowohl Nukleinsäuren als auch andere Analyten nachzuweisen. Dies ermöglicht den automatisierten Nachweis mehrerer diagnostischer Targets, wie beispielsweise Nukleinsäuren, Proteine, Antikörper und Enzyme mittels automatisierter Prozessabläufe. Damit wird auch ein diagnostisches System bereitgestellt, das die Durchführung einer Vielzahl von unterschiedlichen Tests erlaubt. Die Flexibilität bezüglich der Anwendungsvielfalt ist ein bedeutender Vorteil im klinischen Labor.

Gemäß einem ersten Aspekt der Erfindung wird ein Verfahren zum Nachweis und zur Bestimmung der Bindungsstärke wenigstens eines Analyten in einer Probe bereitgestellt, wobei
A) an einer festen Oberfläche, an räumlich definierten Positionen Komplexe a) und b) immobilisiert werden, umfassend
   a) ein oder mehrere Targetmoleküle, an denen der Analyt spezifisch gebunden ist und
   b) ein oder mehrere Referenzanalyten;
   wobei die Komplexe a) und b) mindestens eine Nachweisfluoreszenzmarkierung aufweisen;
B) eine Messung der Nachweisfluoreszenzmarkierung der Komplexe a) und b) erfolgt;
C) eine Dissoziation der Bindung des Analyten vom Targetmolekül sowie der Bindung der Nachweisfluoreszenzmarkierung vom Referenzanalyten und vom Analyten simultan erfolgt;
D) eine Messung der Nachweisfluoreszenz vor und während der Dissoziation ortsaufgelöst an mindestens zwei verschiedenen Messpunkten erfolgt;
E) die Nachweisfluoreszenzverringerung des mindestens einen Referenzanalyten mit der Nachweisfluoreszenzverringerung des mindestens einen Analyten verrechnet wird und die Bindungsstärke des Analyten als indirekt proportionale Abhängigkeit zur Nachweisfluoreszenzverringerung des mindestens einen Analyten ausgedrückt wird.

Gemäß einem zweiten Aspekt wird ein Kit bereitgestellt, zum Nachweis und zur Bestimmung der Bindungsstärke wenigstens eines Analyten in einer Probe enthaltend:
i) wenigstens ein Targetmolekül, an denen der Analyt spezifisch binden kann;
ii) wenigstens einen Referenzanalyten;
iii) eine feste Oberfläche, an welche das Targetmolekül und der Referenzanalyt spezifisch binden kann;
iii) wenigstens eine Nachweisfluoreszenzmarkierung, die wenigstens einen AnalytBinder und wenigstens einen Fluoreszenzfarbstoff umfasst sowie
iv) ein oder mehrere Puffersysteme.

Gemäß einem dritten Aspekt betrifft die vorliegende Erfindung die Verwendung eines automatisierten Systems zum Nachweis und zur Bestimmung der Bindungsstärke wenigstens eines Analyten in einer Probe mittels des oben genannten Verfahrens.

Andere Gegenstände, Merkmale, Vorteile und Aspekte der vorliegenden Anmeldung sind für den Fachmann aus der folgenden Beschreibung und den davon abhängigen Ansprüchen ersichtlich. Es ist jedoch so zu verstehen, dass die folgende Beschreibung, die abhängigen Ansprüche und die spezifischen Beispiele, die sich auf bevorzugte Ausführungsformen der Anmeldung beziehen, nur zur Veranschaulichung angegeben werden. Verschiedene Änderungen und Modifikationen im Rahmen der offenbarten Erfindung ergeben sich für den Fachmann ohne weiteres aus den folgenden Ausführungen.

### Ausführliche Beschreibung der Erfindung

Gemäß einem ersten Aspekt wird ein Verfahren zum Nachweis und zur Bestimmung der Bindungsstärke wenigstens eines Analyten in einer Probe gemäß Anspruch 1 bereitgestellt.

Der Begriff "Nachweis" umfasst die Bestimmung der An- oder Abwesenheit des Analyten in der Probe und erlaubt in Ausführungsformen eine qualitative, semiquantitative oder eine quantitative Bestimmung der Menge des Analyten in einer Probe.

Der Begriff "Bindungsstärke" umfasst Affinität und Avidität, ohne beides zu unterscheiden.

Die einzelnen Schritte des erfindungsgemäßen Verfahrens werden nachfolgend erläutert.

Die Bezeichnung "Targetmolekül" umfasst eine Vielzahl von Molekülen einer Spezies, wobei die Moleküle einer Spezies nicht unbedingt identisch sein müssen, sich aber derselben analytischen Fragestellung spezifisch zuordnen lassen sollten.

Die Bezeichnung "Probe" bezeichnet eine zu untersuchende Zusammensetzung, die für die ex vivo oder in vitro Untersuchung verwendet werden kann. Bei der Probe handelt es sich bevorzugt um biologisches oder medizinisches Material, also Material, das aus einem Organismus, von Bestandteilen eines Organismus oder aus Zellen gewonnen wird. Das Material kann, bevor es als Probe im erfindungsgemäßen Verfahren eingesetzt wird, weiteren Behandlungsschritten unterzogen werden, z.B. um das Material in einen Zustand zu versetzen, in dem es sich als Probe für das erfindungsgemäße Verfahren besonders eignet. Zur Aufbereitung können gängige Methoden verwendet werden, die dem Fachmann bekannt sind. So können beispielsweise bestimmte Stoffe oder Stoffklassen, wie z.B. Proteine, Lipide oder Nukleinsäuren, aus dem Material aufgereinigt werden, welche dann als Probe im erfindungsgemäßen Verfahren eingesetzt werden können.

### A) Immobilisierung der Komplexe a) und b)

Zur Durchführung des erfindungsgemäßen Verfahrens kann die Probe einem Vorbereitungsschritt unterzogen werden. Der Vorbereitungsschritt dient dazu, dass Matrixeffekte aus der Probe keinen nennenswerten Einfluss auf die Sensitivität und Spezifität des Tests haben. Beispielhafte Vorbereitungsschritte umfassen die Probenverdünnung, die Einstellung des pH-Werts oder der lonenstärke des Proben-Inkubationspuffers des Analyten oder der Nachweisfluoreszenzmarkierung bzw. der Waschpuffer. Ein weiteres Beispiel ist die Depletion des Analyten aus der Probe mittels Adsorption oder Affinitätsreinigung. Bevorzugt erfolgt dies durch Affinitätsbindung an Targetmolekülen an der festen Phase des Reaktionsgefäßes. Dadurch ist es möglich, durch nachfolgende Einzelmoleküldetektion den Analyten absolut, d.h. digital, zu quantifizieren.

Beispiele für geeignete Probenmaterialien sind Blut, Serum, *Liquor cerebrospinales,* Fruchtwasser, Urin, Stuhl, Zellen, Geweben, Abwasser, Boden, Mikroorganismen, Viren in denen die Analyten einzeln oder in Kombination in unterschiedlichen Konzentrationen auftreten können.

In der Probe können ein oder mehrere Analyten gelöst vorliegen, die beispielsweise zu den Substanzklassen der Proteine, posttranslational modifizierte Proteine, citrullinierte Proteine, carbamylierte Proteine, phosphorylierte Proteine, alkylierte Proteine, Glykoproteine, Antikörper, Autoantikörper, Antigen, Aptamere, Avidine, Lektine, Enzyme, Hormone, Cytokine, Prionen, Peptide, Kohlenhydrate, Oligosaccharide, Nukleinsäuren, Nukleinsäurenanaloga, Lipide, Phospholipide und Glykolipide gehören. Die Analyten können in der Probe einzeln oder in Kombination in unterschiedlichen Konzentrationen auftreten.

Befinden sich die Analyten in Proben, wie beispielsweise Gewebe oder Zellen, kann es erforderlich sein, diese durch geeignete chemische Fixierungstechniken an Ort und Stelle, d.h. im korrekten morphologischen Kontext zu immobilisieren, wie beispielsweise Histone, Doppelstrang-DNA, Helicasen und Topoisomerasen für den Zellkern, Collagene, Laminin oder Fibronektin für Bindegewebe, Glycosyltransferasen für den Golgi-Aparat bzw. Ganglioside oder neuronale Zelladhäsionsmoleküle für die Nervenzellmembran. Moleküle in Gewebeschnitten können als Targetmolekül fungieren, wenn die Gewebeschnitte zur immunhistochemischen Bestimmung von Antikörpern in Patientenseren eingesetzt werden.

Analyten sind Biomoleküle, die entsprechend der Heterogenität lebender Systeme erheblich in ihren Eigenschaften variieren können. Mutationen oder posttranslationale Modifikationen sind zwei Beispiele, die dazu führen können, dass die Bindung eines Analyten an das spezifische Targetmolekül nicht oder nur schwach stattfindet. Im Umkehrschluss können abweichende Bindungsstärken im erfindungsgemäßen Verfahren ein Hinweis darauf sein, dass verschiedene Formen des Analyten in der Probe vorliegen. Beispiele für verschiedene Formen von Analyten in der Probe sind unterschiedliche Antikörperpopulationen, die sich gegen verschiedene Epitope eines Antigens richten oder somatische Mutationen bzw. Methylierungen in genomischer DNA.

In einer bevorzugten Ausführungsform der Erfindung können die Analyten vor, während oder nach ihrer Bindung an das jeweilige Targetmolekül chemisch, enzymatisch und /oder physikalisch modifiziert werden. Eine chemische Modifizierung von Nukleinsäuren könnte beispielsweise die Bisulfit-Konversion von Cytosin zum Nachweis von Methylgruppen sein. Proteine können beispielsweise zur Blockierung von SH-Gruppen alkyliert oder durch Aldehyde fixiert werden. Kohlenhydrate können beispielsweise im Bereich vicinaler OH-Gruppen durch Metaperiodat oxidiert werden. Die enzymatische Veränderung von Nukleinsäuren kann beispielsweise durch Restriktasen oder Ligasen, von Proteinen durch Proteasen oder durch posttranslational modifizierende Enzyme wie z.B. Peptidyl-Argin-Deiminase, Glykosidasen oder Glycosyltransferasen und von Kohlenhydraten ebenso durch Glycosidasen erfolgen. Beispiele für die physikalische Modifizierung von Analyten sind deren Erhitzung, Gefrierung, Gefriertrocknung, Ultraschallbehandlung, Gammabestrahlung oder UV-Bestrahlung.

In einer ebenfalls bevorzugten Ausführungsform der Erfindung, kann die proteolytische Spaltung der Analyten im Falle von Antikörpern mit spezifischen Proteasen wie z.B. Pepsin zur gezielten Fragmentierung der Antikörper erfolgen. Das hat den Vorteil, dass eine gezielte Beeinflussung der Bindungseigenschaften der Antikörper möglich ist. In diesem Falle können die entstehenden Fab-Fragmente der Antikörper als monovalent an das jeweilige Targetmolekül binden oder gebundene Analyten der Untersuchung ihrer Bindungseigenschaften zugeführt werden. In der Regel verringert sich dadurch die Bindungsstärken der gebundenen und danach fragmentierten Antikörper. Als Konsequenz verringern sich die Schmelztemperaturen der verbliebenen monovalenten Fab-Fragmente, so dass auch sehr stark affine Antikörper einer Untersuchung zugeführt werden können. Damit wird das Problem gelöst, dass stark affine Antikörper über den gesamten Temperaturbereich nicht dissoziiert werden können und so nicht sicher von unspezifischen Bindungen der Nachweisfluoreszenzmarkierung oder anderer Probenbestandteile abgegrenzt werden können. Bei der Detektion fragmentierter Antikörper ist zu beachten, dass die eingesetzte Nachweisfluoreszenzmarkierungen über den Analyt-binder ausreichend stark an Fab-Fragmente bindet.

Als feste Oberflächen für die Anbindung und Immobilisierung der Komplexe a) und b) können planare feste Phasen eingesetzt werden, die feste Oberflächen von Trägermaterialien sein können, wie beispielsweise die Oberfläche eines Reaktionsraumes, eines "Wells" einer Mikrotiterplatte, eines Partikels oder ähnliches. Bevorzugt sind die festen Oberflächen transparent und zeichnen sich durch eine geringe Autofluoreszenz aus, wie beispielsweise Polycarbonat, Polysterol, Polypropylene, Quarzglas oder Glas. Dies ermöglicht eine fluoreszenzoptische, bildgebende Detektion im Durchlicht oder bei intransparenten Trägermaterialien im Auflicht.

In einer bevorzugten Ausführungsform der Erfindung sind feste Oberflächen planare, transparente Oberflächen eines Trägermaterials ausgewählt aus der Gruppe Objektträger, Biochip, Fluidzelle, Mikrotestplatte, 8er-Modul einer Mikrotestplatte oder Einzelkavität einer "break apart"-Mikrotestplatte. Bevorzugt sind Objektträger oder Mikrotestplatten. Dies hat den Vorteil, dass dafür bereits verschiedenste Automatisierungslösungen für die Abarbeitung von Tests mit mehreren verschiedenen Inkubationsschritten existieren.

In einer besonders bevorzugten Ausführungsform umfasst der Reaktionsraum einen planaren mikroskopierbaren Boden mit geringer Autofluoreszenz als Trägermaterial und kann direkt oder vermittelt über Oberflächenfunktionalisierungen, fluoreszenzkodierte Beads, histologische Gewebeschnitte oder Zellen nach physikalischer oder chemischer bzw. ohne physikalische oder chemische Fixierung Targetmoleküle binden.

Der Reaktionsraum kann mit geeigneten Deckeln oder Verschlussfolien abgedeckt oder mit Öl, beispielsweise Mineralöl, überschichtet werden, um eine Verdunstung der Flüssigkeit aus dem Reaktionsraum zu vermeiden. Im Falle von Objektträgern können entsprechende Deckglassysteme aus der Histologie eingesetzt werden, die auch den Spalt zwischen Deckglas und Objektträger seitlich verschließen.

Bevorzugt sind die festen Oberflächen der Trägermaterialien so funktionalisiert, dass eine permanente Immobilisierung der Komplexe a) und b) möglich ist. Beispiele für solche Oberflächenfunktionalisierungen sind Carbox-, Amino-, Sulfhydryl-, Epoxy-, Aldehydgruppen, Metallbeschichtungen wie Gold oder Nickel, aber auch Polymere wir Polyacrylsäure, Polylysin, aktiviertes Polyethylenglycol oder Nanozellulose. Somit ist es möglich, dass an die innere mikroskopierbare Oberfläche der Trägermaterialien, die später mit der Probe benetzt wird, die Targetmoleküle und Referenzanalyten an den Oberflächenfunktionalisierungen direkt oder vermittelt über fluoreszenzkodierte Beads, histologische Gewebeschnitte oder Zellen nach physikalischer oder chemischer bzw. ohne physikalische oder chemische Fixierung, kovalent oder nichtkovalent gebunden werden können.

An der festen Oberfläche sind erfindungsgemäß, an räumlich definierten Positionen Komplexe a) und b) immobilisiert, wobei der Komplex a) ein oder mehrere Targetmoleküle, an denen der Analyt spezifisch gebunden ist und der Komplex b) ein oder mehrere Referenzanalyten umfasst.

In einer Ausführungsform der Erfindung können die Komplexe a) und b) getrennt voneinander oder im Gemisch in definierten zweidimensionalen Positionen an der festen Oberfläche des Trägermaterials punkt- oder linienförmig gebunden oder an fluoreszenz- und/oder größenkodierten Mikropartikeln, zufällig räumlich verteilt gebunden werden oder in histologischen Gewebeschnitten oder in Zellen bereits gebunden vorliegen.

Die Komplexe a) und b) können beispielsweise sehr dicht bis konfluierend, eine Mono-Layer bildend, immobilisiert werden. In einer bevorzugten Ausführungsform wird die Immobilisierung der Komplexe a) und b) in einem Abstand vorgenommen werden, der groß genug ist, um einzelne Moleküle bei molekularer Auflösung des Detektionssystems auch bei Sättigung aller Analytbindungsstellen aufzeichnen zu können.

Als Targetmoleküle können Biomoleküle eingesetzt werden, die eine intermolekulare Wechselwirkung mit den Analyten eingehen können. Bevorzugte Targetmoleküle gehören beispielsweise zu den Substanzklassen der Proteine, posttranslational modifizierte Proteine, citrullinierte Proteine, carbamylierte Proteine, phosphorylierte Proteine, alkylierte Proteine, Glykoproteine, Antikörper, Antigene, Autoantigene, Aptamere, Avidine, Lektine, Enzyme, Hormone, Cytokine, Prionen, Peptide, Kohlenhydrate, Oligosaccharide, Ganglioside, Nukleinsäuren, Nukleinsäurenanaloga, Lipide, Phospholipide und Glykolipide und können aus natürlichen Quellen gereinigt, synthetisiert oder rekombinant hergestellt werden. Die Targetmoleküle können unmodifiziert oder modifiziert vorliegen. Beispiele für geeignete Modifizierungen sind die Biotinylierung, Carboxylierung oder Alkinierung oder auch gewobbelte Peptidsequenzen oder posttranslational veränderte Peptdiseqeunzen oder modifizierte Oligosaccharide, wie beispielsweise sulfatierte, oxydierte oder methylierte Monosaccharidbausteine in Oligosacchariden. Bevorzugt werden Antigene, Autoantigene, Antikörper, Oligosaccharide, Nukleinsäuren als Targetmoleküle eingesetzt.

In einer bevorzugten Ausführungsform werden zusätzlich Targetmoleküle für die Immobilisierung verwendet, die keinen der spezifischen Analyten binden. Diese können als Negativkontrolle dienen.

Die weitgehend stabile Bindung der Targetmoleküle zur direkten oder indirekten Immobilisierung an der festen Oberfläche kann durch kovalente und nicht kovalente Bindung erfolgen. Die kovalente Bindung kann beispielsweise durch Carbodiimid-Kopplung oder Azid-Alkin-Kupfer-vermittelte Cycloaddition, der Klick-Chemie, hergestellt werden, bevorzugt ist die Carbodiimid-Kopplung. Ein Beispiel für eine geeignete nichtkovalente Kopplung ist die Avidin-Biotin-Kopplung. Bevorzugt erfolgt die kovalente Kopplung der Targetmoleküle.

Die Bindung der Targetmoleküle an die Analyten ist ebenso kovalent und nichtkovalent möglich, wobei die nichtkovalente Bindung, wie beispielsweise van der Waals-Kräfte, hydrophobe Wechselwirkungen oder lonenbeziehungen, bevorzugt ist.

Ein Beispiel für die kovalente Bindung von Analyten an das Targetmolekül können Antikörper gegen Proteinantigene sein, die mit ihrem Paratop an das Epitop des Protein-Antigens binden. Sulfhydryl-Gruppen in Paratop oder Epitop können spontan kovalente Disulfidbindungen ausbilden, so dass die Bindung des Antikörpers nur unter reduzierenden Bedingungen, die zur Auflösung von Disulfidbrücken führen, vom entsprechenden Antigen losgelöst werden können.

An der festen Oberfläche kann der Komplex b), umfassend einen oder mehrere Referenzanalyten, direkt oder indirekt gebunden sein. Der Referenzanalyt ist im Gegensatz zu den Targetmolekülen in der Regel nicht dazu bestimmt, Analyten aus der Probe zu binden.

Als Referenzanalyten können beispielsweise Moleküle der Substanzklassen der Proteine, posttranslational modifizierte Proteine, citrullinierte Proteine, carbamylierte Proteine, alkylierte Proteine, phosphorylierte Proteine, Glykoproteine, Antikörper, Autoantikörper, Antigen, Aptamere, Avidine, Lektine, Enzyme, Hormone, Cytokine, Prionen, Peptide, Kohlenhydrate, Oligosaccharide, Nukleinsäuren, Nukleinsäurenanaloga, Lipide, Phospholipide und Glykolipide, eingesetzt werden.

Die Immobilisierung der Referenzanalyten und der Targetmoleküle kann beispielsweise im Reaktionsgefäß ortskodiert oder bei Verwendung von Beadassays fluoreszenz- und/oder größenkodiert erfolgen. Ebenfalls können die Referenzanalyten ortskodiert immobilisiert sein und die Targetmoleküle können z.B. über fluoreszenz- und/oder größenkodierte Beads immobilsiert sein.

Beispielsweise ist es ebenfalls möglich, dass die Targetmoleküle im Singleplex zufällig verteilt an der festen Phase gebunden oder im Multiplexformat ortskodiert und die Referenzanlyten auf der Oberfläche von nichtkodierten Beads im Singleplex bzw. auf fluoreszenz- und/oder größenkodierten Beads im Multiplexformat immobilisiert werden. Alternativ können die Referenzanlyten im Singleplex zufällig verteilt an der festen Phase gebunden oder im Multiplexformat ortskodiert und die Targetmoleküle auf der Oberfläche von nichtkodierten Beads im Singleplex oder auf fluoreszenz- und/oder größenkodierten Beads im Multiplexformat immobilisiert werden.

In einer bevorzugten Ausführungsform sind Targetmolekül und Referenzanalyt an fluoreszenz- und/oder größenkodierten Beads gebunden und die Beads sind zufallsverteilt oder geordnet verteilt an die feste Oberfläche des Trägermaterials permanent immobilisiert.

In einer Ausführungsform der Erfindung, kann sich der Referenzanalyt und der Analyt molekular unterscheiden. Während der Analyt beispielsweise IgG aus humanem Serum sein kann, reicht es aus, wenn als Referenzanalyt eine Präparation des Fc-Teils von humanem IgG oder ein spezifisches IgG-Peptid dient, sofern die Nachweisfluoreszenzmarkierung Anti-Fc-human-IgG-Fluoreszenzkonjugat ist, welches gleichermaßen oder bevorzugt vergleichbar stark an den Fc-Teil des Analyten und den Referenzanalyten bindet.

Im Schritt A) des erfindungsgemäßen Verfahrens können die Komplexe a) und b) erhalten werden, indem die feste Oberfläche des Trägermaterials mit Targetmolekülen und Referenzanalyt beschichtet wird. In einer Ausführungsform können dabei Targetmoleküle mit verschiedenen Bindungseigenschaften und Referenzanalyten getrennt voneinander oder im Gemisch in definierter zweidimensionaler Anordnung an der festen Oberfläche des Reaktionsgefäßes punkt- oder linienförmig verteilt immobilisiert werden. In einer bevorzugten Ausführungsform sind Targetmoleküle und Referenzanalyt an fluoreszenz- und/oder größenkodierten Mikropartikeln, zufällig räumlich verteilt gebunden oder liegen in histologischen Gewebeschnitten oder in Zellen gebunden vor.

Es folgt die Zugabe der Probe und die erste Inkubation a), in der der Analyt am Targetmolekül binden kann. Die Probe kann dazu mit einem wässrigen Reaktionspuffer verdünnt werden oder unverdünnt für die erste Inkubation eingesetzt werden.

Als Reaktionspuffer, sind beispielsweise Phosphatpuffer, Phosphat-Citrat-Puffer, Tris-HCl-Puffer, Tris-Acetat-Puffer, Carbonatpuffer, HCl-KCl-Puffer, HEPES-Puffer, Glycin-HCL-Puffer, MES-, MOPS-, PIPES-Puffer oder Tricine-HCl-Puffer geeignet. Die Molarität des wässrigen Reaktionspuffers kann zwischen 0,01 bis 0,5 M liegen.

Der pH-Wert der Probe kann zwischen pH 5 und pH 9 liegen. Die Probe kann ebenfalls Detergenzien, wie beispielweise Tween^{®} 20 (Polyoxyethylen-20-sorbitanmonolaurat), Triton^{®} X100 (p-*tert*-Octylphenoxy)polyethoxyethanol), Nonident P40 (Octylphenoxypolyethoxyethanol), Octyl-beta-Glycosid enthalten. Die Inkubationszeit kann zwischen 5 Minuten bis 72 Stunden liegen und sollte zur Sicherstellung einer hohen Reproduzierbarkeit bevorzugt bis zur Einstellung des Bindungsgleichgewichtes, der sogenannten Plateauphase der Bindung oder der Sättigung der Bindung führen.

In einer bevorzugten Ausführungsform der Erfindung enthält der Reaktionspuffer 0,05 M Natrium-Phosphatpuffer mit Zusatz von 0,9% NaCl und 0,05 % Tween^{®} 20, mit einem pH-Wert zwischen 7 und 8, bevorzugt ist PBS-T. Zusätzlich können im Reaktionspuffer noch Blockierungssubstanzen, wie beispielsweise Serumalbumin, Casein oder Polyvinylpyrolidon enthalten sein, um unspezifische Bindungen des Analyten zu verringern.

In einer weiteren bevorzugten Ausführungsform der Erfindung erfolgt mindestens ein Waschschritt (Inkubation b)) der Komplexe a) und b) mit einem Waschpuffer vor der Inkubation c) mit der Nachweisfluoreszenzmarkierung. Dieser Schritt kann nach Abschluss der ersten Inkubation a) der Probe und Bindung des Analyten an das Targetmolekül erfolgen. Bevorzugt erfolgt die Inkubation b) mit dem Waschpuffer mehrmalig, beispielsweise 1- bis 6-mal, bevorzugt 1- bis 3-mal, besonders bevorzugt 3-mal. Durch den Waschschritt nach der Bindung des Analyten an das Targetmolekül, kann die Probenmatrix aus dem Reaktionsraum entfernt werden, was die Komplexität der Beeinflussung der Bindung des Analyten an sein Targetmolekül während der Dissoziation weiter verringert.

Geeignete Waschlösungen sind wässrige Pufferlösungen mit einer Molarität zwischen 0,01 bis 0,5 M, wie zum Beispiel Phosphatpuffer, Phosphat-Citrat-Puffer, Tris-HCl-Puffer, Tris-Acetat-Puffer, Carbonatpuffer, HCl-KCl-Puffer, HEPES-Puffer, Glycin-HCL-Puffer, MES-, MOPS-, PIPES-Puffer, Tricine-HCl-Puffer. Es ist aber auch möglich, Wasser als Reaktionslösung einzusetzen. Die Inkubation der verdünnten Nachweisfluoreszenzmarkierung erfolgt bei einem pH-Wert zwischen pH 5 und pH 9 unter Zusatz von Detergenz wie zum Beispiel Tween 20, Triton X100, Nonident P40, Octyl-beta-Glycosid in Abhängigkeit der Fragestellung für 5 Minuten bis 72 Stunden. Bevorzugt dient PBS-T als Waschpuffer.

In einer bevorzugten Ausführungsform unterscheidet sich der Waschpuffer vom Reaktionspuffer, beispielsweise in lonenstärke oder pH-Wert. Die lonenstärke des Waschpuffers liegt zwischen 0,05 und 0,5M und der pH-Wert zwischen 6 bis 9. Dies ermöglicht unspezifisch gebundene Probenbestandteile aus dem Reaktionsraum herauswaschen zu können. Wasch- und Reaktionspuffer können aber auch identisch sein.

In einer bevorzugten Ausführungsform erfolgt ein weiterer Inkubationsschritt c) mit einer Nachweisfluoreszenzmarkierung. Hierdurch erfolgt die Bindung der Nachweisfluoreszenzmarkierung an die Analyten und Referenzanalyten. Die Nachweisfluoreszenzmarkierung für die Bindung an die Analyten und die jeweiligen Referenzanalyten der Komplexe a) und b) kann eine vergleichbare Stärke und Spezifität für die Bindung aufweisen. Ebenfalls kann das Dissoziationsverhalten für den Referenzanalyten bereits bekannt sein. Die Fluoreszenzmarkierung des Analyten kann dabei vor, während oder auch nach der Bindung des Analyten an ein Targetmolekül erfolgen.

In einer alternativen Ausführungsform, kann der Analyt oder auch der Referenzanalyt selbst bereits eine Fluoreszenzmarkierung aufweisen, wodurch ein weiterer Inkubationsschritt c) mit einer Nachweisfluoreszenzmarkierung entfällt.

In einer bevorzugten Ausführungsform der Erfindung umfasst die Nachweisfluoreszenzmarkierung einen Analyt-Binder und einen Fluoreszenzfarbstoff. Besonders bevorzugt umfasst die Nachweisfluoreszenzmarkierung einen Analyt-Binder, einen Fluoreszenzfarbstoff und einen Spacer zwischen Analyt-Binder und Fluoreszenzfarbstoff.

Geeignete Analyt-Binder können Moleküle mit mindestens einer Bindungsdomäne für einen oder mehrere Analyten sein, wie beispielsweise Analyt-spezifische Antikörper. Ein IgG-Antikörper zeichnet sich dabei durch zwei Bindungsstellen, ein IgA-Antikörper durch vier und ein IgM-Antikörper durch 10 identische Bindungsstellen, aus. Ein weiteres Beispiel sind enzymatisch aus Antikörpern hergestellte Fab-Fragmente, die zwei oder nur eine Bindungsstelle aufweisen können. Ebenfalls geeignete Analyt-Binder können gentechnisch hergestellte Antikörper, die sogenannten rekombinanten Antikörper, sein, wie beispielsweise Nanobodies oder bispezifische Antikörper, die zwei verschiedene Bindungsstellen für verschiedene Analyten aufweisen.

In einer Ausführungsform kann der Analyt-Binder ausgewählt sein aus der Gruppe der biotinylierten Antikörper, rekombinanten Antikörper, biotinylierten Nukleinsäuren, Fab-Fragmente, Avidin, Nickel-NTA, Protein A und Protein G, DNA, Lektine, Aptamere, Nukleinsäureanaloga oder Nukleinsäuren, bevorzugt ist Avidin, Anti-Immunglobulin-Antikörper und Antikörper mit Spezifität für einen oder mehrere Analyten der Analyt-Binder. In einer besonders bevorzugten Ausführungsform der Erfindung werden thermisch besonders stabile Antikörper als Analytbinder eingesetzt. Beispiele sind Antikörper von kamelartigen Säugetieren oder Haien sowie von gentechnisch veränderten und rekombinant hergestellt Antikörpern, die eine Temperaturstabilität von über 90 °C haben können.

In einer bevorzugten Ausführung der Erfindung ist Avidin der Analyt-Binder, welches an Biotin-Reste biotinylierter oder bis-biotinylierter Analyten oder an biotinähnliche Aminosäuresequenzmotive bindet. Die Bindung der Analyt-Binder an den Analyten erfolgt bevorzugt nichtkovalent z.B. nach Biotinylierung oder bis- Biotinylierung des Analyten durch Bindung von fluoreszenzmarkiertem Avidin. Die Bindung von Bis-Biotin an Avidin ist besonders thermostabil und deshalb eine bevorzugte Ausführungsform der Erfindung.

Als Analyt-Binder können auch biotinylierte Antikörper eingesetzt werden, die spezifisch an den Analyten humanes IgG binden. Nach Entfernung des ungebundenen Überstandes wird Avidin für einen weiteren Inkubationsschritt d) zum Testansatz gegeben, sodass dieses an die Biotinreste des biotinylierten Antikörpers, binden kann. Im nachfolgenden Inkubationsschritt d) binden an die noch freien Biotin-Bindungsstellen des gebunden Avidins jeweils ein oder im Gemisch verschiedene biotinylierte Fluoreszenzfarbstoffe. Es ist zudem möglich, Avidin vor Zugabe zum Testansatz kovalent an den Analytbinder zu koppeln ebenso wie die biotinylierten Fluoreszenzfarbstoffe. Die biotinylierten Fluoreszenzfarbstoffe können dabei monomolekular vorliegen oder im multimolekularen Komplex kovalent gebunden an z.B. biotinylierten Poly-L-Lysin oder Polyethylenglycol.

Alternativ können biotinylierte Nukleinsäuren, die fluoreszenzmarkiert sind oder die unmarkiert als Primer für eine isothermale Nukleinsäureamplifikation dienen, gebunden werden. Die fluoreszenzmarkierten Sonden können sofort oder nach Hybridisierung weiterer markierter Oligonukleotidsonden direkt detektiert werden. Im Falle der Bindung unmarkierter Oligonukleotide und isothermaler Amplifikation wird immobilisiertes Amplifikat gebildet, an das bevorzugt simultan zur Bildung markierte Sonden hybridisieren. Beispiele für isothermale Amplifikationen sind Loop Mediated Amplification (LAMP), Helicase Dependent Amplification (HDA), Rolling Circle Amplification (RCA) und Strand Displacement Amplification (SDA). Die isothermale Amplifikation ist vom Fachmann so zu gestalten, dass das Amplifikat an der festen Phase immobilisiert am Ort der Bildung verbleibt und fluoreszierende Spots mit einem Durchmesser von 0,5 µm bis 6 µm bilden. Bevorzugt haben die Spots im Mittel Durchmesser zwischen 1 µm bis 3 µm. Besonders bevorzugt ist die RCA, bei der als Amplifikat ein fadenförmiges Molekül aus repetitiven Sequenzen entsteht, an welche viele fluoreszenzmarkierte Sonden binden können. Pro Avidin-Molekül können noch bis zu drei Primer binden und durch RCA vervielfältigt werden, wobei die drei Fäden einen gemeinsamen Spot bilden.

Alternativ können Primer für die isothermale Amplifikation direkt, zum Beispiel an den AnalytBinder kovalent gekoppelt werden, wodurch Inkubationsschritte gespart werden.

Ebenfalls geeignete Analyt-Binder sind beispielsweise Nickel-NTA zur Bindung an HIS-Tag-Proteine, Protein A und Protein G für die Bindung an Immunglobuline, DNA für die Bindung an DNA-bindende Proteine, Lektine für die Bindung von Kohlenhydraten und Glycolipiden, Oligonukleotide gefertigt als Nukleinsäureanaloga oder Nukleinsäuren an DNA oder RNA.

Als Fluoreszenzfarbstoffe können solche eingesetzt werden, die stabil gegenüber thermischen Einflüssen, pH-Änderungen und/oder Änderungen der lonenzusammensetzung des Inkubationspuffers sind. Bevorzugte Farbstoffe sind beispielsweise Verbindungen aus den Substanzklassen der Coumarine, Rhodamine, Fluoresceine, Quantum-Dots, Phycobiliproteine, fluoresziierende Proteine, Bodipy-Farbstoffe, Nilrot, Acridifarbstoffe, Sybrgreen, Mito-Tracker-Farbstoffe, Merocyanin-Farbstoffe, besonders bevorzugt sind Fluorescein, Atto647N (**SEQ ID NO. 3,** Atto-Tec GmbH) und Rhodamin 6G, S2381 (FEW GmbH).

Geeignete Spacer zwischen Analyt-Binder und Fluoreszenzfarbstoffen können unterschiedlicher molekularer Struktur sein und dienen dazu, den Abstand der Fluoreszenzfarbstoffe zu den Analyt-Bindern zu vergrößern. Dies soll verhindern, dass zum einen die Bindung der Analyt-Binder an den Analyten sterisch behindert wird und andererseits, Quenching-Effekte durch molekulare Interaktionen die Helligkeit der Emission der Fluoreszenzfarbstoffe verschlechtern.

Beispiele für geeignete Spacer sind Amino-Alkyl-Spacer oder Amino-Polyethylenglycol-Spacer. Die Spacer werden bevorzugt kovalent an den Analyt-Binder bzw. an den Fluoreszenzfarbstoff z.B. mittels Carodiimid-Kopplung oder Click-Chemie gebunden. Ebenso ist es möglich, dass die Bindung der Fluoreszenzfarbstoffe an den Analyt-Binder nichtkovalent erfolgt, wie z.B. die Bindung von SybrGreen an Nukleinsäuren.

In einer bevorzugten Ausführungsform der Erfindung bindet der Analyt-Binder kovalent über einen Spacer an Avidin, so dass bis zu vier Bindungsstellen für biotinylierte bzw. bisbiotinylierte Fluoreszenzfarbstoffe oder Primer für die isothermale Amplifikation frei bleiben.

In einer besonders bevorzugten Ausführungsform der Erfindung wird der Fluoreszenzfarbstoff mit einem Biotin-Alkyl-Spacer an Avidin gebunden, welches bereits eine Biotingruppe des Analyten gebunden hat. Dadurch ist es möglich, dass bis zu drei biotinylierte Fluoreszenzfarbstoffe an Avidin binden können.

Besonders bevorzugt wird als Nachweisfluoreszenzmarkierung Anti-human-lgG-Atto647N-Konjugat oder Anti-human-lgG-Biotin-Konjugat eingesetzt.

Die Nachweisfluoreszenzmarkierung des Analyten kann aber auch direkt ohne Spacer durch kovalente Kopplung eines Fluoreszenzfarbstoffes, beispielsweise mittels SNAP-Tag, erfolgen.

Ebenfalls möglich ist, dass der Analyt eine Autofluoreszenz aufweist, die durch molekulare Chimerisierung eines Analyten mit einem fluoreszierenden Protein und rekombinanter Expression erreicht werden kann, sofern es sich bei Analyten um ein Peptid oder Protein handelt. Ein weiteres Anwendungsbeispiel ist die Verwendung von fluoreszenzmarkierten Antikörpern aus Hyperimmunseren die spezifisch an den Analyten binden.

Ungebundene Nachweisfluoreszenzmarkierung kann durch nochmaliges Waschen mit dem Waschpuffer entfernt werden, wonach die Messung der Nachweisfluoreszenz erfolgen kann. Bevorzugt wird dazu der gleiche Waschpuffer wie zuvor beschrieben eingesetzt.

### B) Messung der Nachweisfluoreszenzmarkierung

In einer möglichen Ausgestaltung der Erfindung, erfolgt die Messung der Nachweisfluoreszenz ortsaufgelöst, da die Nachweisfluoreszenz für mindestens einen Analyten und die Referenzfluoreszenz bestimmt werden kann.

Um die Nachweisfluoreszenz für mehrere Analyten und Referenzanalyten durch simultane ortsaufgelöste Messung (multiplex) differenzieren zu können, werden in einer weiteren Ausführungsform der Erfindung die Targetmoleküle und die Referenzanalyten an verschiedenen Positionen der festen Phase desselben Reaktionsansatzes immobilisiert. Dem Fachmann sind verschiedene Multiplex-Detektionssysteme bekannt, die dafür eingesetzt werden können, mehrere Analytenmoleküle und entsprechende Referenzmoleküle in einem Reaktionsansatz simultan nachzuweisen, wie z.B. Biochips, Dot-ELISA, Suspensions-Beadassays oder Festphasen-Beadassays. In einer bevorzugten Ausführungsform der vorliegenden Erfindung erfolgt somit die Nachweisfluoreszenzmarkierung mit einem Multiplex-Detektionssystem, ausgewählt aus der Gruppe Biochips, Dot-ELISA, Suspensions-Beadassays oder Festphasen-Beadassays, besonders bevorzugt werden Festphasen-Beadassays (Solid-phase-Bead-Assay) eingesetzt.

Bei der Verwendung ortskodierter Biochips werden als feste Oberfläche planare Objektträger eingesetzt, auf denen linienförmig oder punktförmig die Targetmoleküle z.B. aufgedruckt oder aufgesprüht werden können. Aus der zweidimensionalen Anordnung der Targetmoleküle lässt sich durch Detektion und Auswertung erkennen, welches Targetmolekül einen Analyten gebunden hat. Durch Einsatz der zuvor beschriebenen Nachweisfluoreszenzen in Kombination mit isothermaler Amplifikation ist es möglich, jedes Molekül einer Molekülspezies mit einer fluoreszenzmikroskopisch erkennbaren Fluoreszenzmarkierung zu versehen und damit nachzuweisen bzw. nachfolgend dessen Bindungsstärke zu bestimmen.

Bei Verwendung fluoreszenz- und größenkodierter Beads in Beadassays werden die Targetmoleküle an Polymerpartikeln, den Beads, gebunden. Jedes Targetmolekül bzw. Targetmolekülgemisch wird auf Beads einer Beadsorte mit spezifischer Kodierung gebunden. Die Kodierung der Beadsorten erfolgt durch Einschluss eines oder mehrerer Fluoreszenzfarbstoffe in spezifischer Konzentration oder das Mischungsverhältnis zueinander sowie der spezifischen Beadgröße. Der Einschluss der Fluoreszenzfarbstoffe kann während bzw. nach der Beadsynthese nichtkovalent oder kovalent erfolgen. Ein und dieselbe Fluoreszenzkodierung kann auf Beadsorten unterschiedicher Größe angewendet werden, was die Zahl verschiedener Kodierungen und damit den Multiplexgrad erhöht. Die mit den Targetmolekülen beschichteten Beads unterschiedlicher Beadsorten werden gemischt und in den Reaktionsraum gegeben, wo sie beim Suspensions-Beadassay in Suspension oder partiell immobilisiert mit dem Analyten interagieren können.

In einer bevorzugten Ausführungsform der Erfindung wird ein Solid-Phase-Beadassay verwendet, wobei die Beads permanent an der festen Phase immobilisiert werden können. Durch Dekodierung der Fluoreszenzkodierung und/oder der Größenkodierung kann bei der Auswertung zugeordnet werden, an welches Targetmolekül ein Analyt gebunden hat.

Der Nachweis des Analyten gilt dann als gesichert, wenn der gemessene Fluoreszenzwert der für den jeweiligen Analyten gemessen wurde, höher ist als ein definierter Schwellwert, der Cut-off.

Der Nachweis des Vorhandenseins eines Analyten in einer untersuchten Probe setzt voraus, dass eine Nachweisfluoreszenz einem spezifisch an ein Targetmolekül gebundenen Analyten zugeordnet werden kann. Um die Spezifität einer Reaktivität im selben Reaktionsansatz sicherzustellen, können interne Negativkontrollen in den Test integriert werden. Dabei handelt es sich bevorzugt um Targetmoleküle, die im Reaktionsraum parallel zu spezifischen Targetmolekülen immobilisiert werden, keinen Analyten binden und deshalb auch keine Nachweisfluoreszenzmarkierung nach Testdurchführung aufweisen. Durch unspezifische Bindungen von Analyten oder anderen Probenbestandteilen können solche internen Negativkontrollen aber auch positiv reagieren, das heißt im wesentlichen Umfang Nachweisfluoreszenzmarkierung aufweisen. Dann ist es möglich, die Bestimmung der Probe als nicht valide zu beurteilen. Alternativ ist es möglich, den relativen Fluoreszenzwert der Negativkontrollen von allen relativen Fluoreszenzmesswerten anderer Targetmoleküle und des Referenzanalyten zu subtrahieren. Eine weitere Möglichkeit zur Erreichung einer hohen Spezifität ist es, einen Cut-off für jedes Targetmolekül zu bestimmen, der überschritten werden muss, um den Messwert als positiv bewerten zu können. Ebenfalls möglich ist die Mitführung einer externen Negativkontrolle, bei der eine für den/die Analyten negative Probe im parallelen Probenansatz in der Probenserie mitgeführt wird, auf die alle anderen Proben der Probenserie referenziert werden.

Ebenso können interne und externe Positivkontrollen genutzt werden, um die Funktionalität des Testsystems während der Testdurchführung zu belegen. Besondere Bedeutung kommt dabei der Positivkontrolle zu, die als Referenzanalyt genutzt werden kann, da dieser als Referenz für die Bindung der Nachweisfluoreszenzmarkierung an den Analyten dient. Ist die Bindung der Nachweisfluorezenz an den Analyten unter den Dissoziationsbedingungen bekanntermaßen irreversibel, weitgehend irreversibel oder findet in immer gleicher Weise statt, kann auf die Referenzierung auch verzichtet werden.

An den Referenzanalyten muss die Nachweisfluoreszenzmarkierung binden, d.h. die Positionen im Reaktionsansatz an denen der Referenzanalyt direkt oder indirekt immobilisiert wurde, reagiert immer positiv und zeigt somit die Funktion des Tests an.

Alternativ ist es möglich, einen Referenzanalyten an der festen Oberfläche zu immobilisieren, der bei Normalfunktion des Tests nicht von der Nachweisfluoreszenzmarkierung gebunden wird, wie zum Beispiel humanes Serumalbumin in einem Antikörpernachweis. Reagiert dieser Referenzanalyt nach Kontaktierung mit der Probe und der Nachweisfluoreszenzmarkierung positiv, kann dies im Sinne einer Negativkontrolle eine unspezifische Bindung von Probenbestandteilen anzeigen.

Erfindungsgemäß sind diese Kontrollen in jeden Reaktionsansatz integriert (siehe Schritt A) und werden deshalb auch als interne Kontrollen bezeichnet.

### C) Dissoziation

Im erfindungsgemäßen Schritt C) erfolgt die Dissoziation der Bindung des Analyten vom Targetmolekül sowie der Bindung der Nachweisfluoreszenzmarkierung vom Referenzanalyten simultan.

In einer bevorzugten Ausführungsform erfolgt die Dissoziation chemisch und/oder physikalisch.

Der Komplex a) mit der Nachweisfluoreszenzmarkierung kann beispielsweise nach Überschichtung mit einem Dissoziationspuffer von den Targetmolekülen losgelöst werden, um sich im Reaktionsraum, der mit Dissoziationspuffer gefüllt ist, verteilen zu können. Durch den damit einhergehenden Verdünnungseffekt verringert sich die Intensität der am Analyten gebundenen Nachweisfluoreszenzmarkierung umgekehrt proportional zur Bindungsstärke des Analyten an das jeweilige Targetmolekül. Die Bindung der Targetmoleküle an die feste Phase und die Bindung der Nachweisfluoreszenzmarkierung sollte bevorzugt möglichst stark sein, um unter den physikochemischen Bedingungen der Dissoziation von Targetmolekül und Analyt sich nicht oder in einem bekannten und bevorzugt geringem Maß zu lösen.

Da Analyt und Referezanalyt hinsichtlich der Bindung der Nachweisfluoreszenzmarkierung dieselben oder sehr vergleichbare Eigenschaften haben, ist es möglich, das Bindungsverhalten des Analyten auf das Bindungsverhalten des Referenzanalyten zu referenzieren. Diese Referenzierung ermöglicht es, den Messfehler zu verringern, der durch die Dissoziation der Bindung des Analyten an die Nachweisfluoreszenzmarkierung entsteht, da auch die Bindung des Referenzanalyten an die Nachweisfluoreszenz in vergleichbarer Weise von der Dissoziation betroffen ist. Dieser Umstand muss bei der Beurteilung der Verringerung der Nachweisfluoreszenz an den Targetmolekülen berücksichtigt oder auch rechnerisch korrigiert werden. Darüber hinaus kann durch die Referenzierung das erfindungsgemäße Verfahren auf verschiedenste Testprinzipien, wie beispielsweise die Beadassays, Biochips, Histologie oder Cytologie und Einzelmolekül-Immunoassay übertragen werden.

In einer Ausführungsform erfolgt die chemische Dissoziation durch Zugabe eines Dissoziationspuffers. Der Dissoziationspuffer dient dazu, gebundenen Analyten gezielt von seiner Bindung an das Targetmolekül zu lösen oder diese Ablösung zu unterstützen. Bei der chemischen Dissoziation sowie bei einer Kombination von chemischer und physikalischer Dissoziation, kann die Dissoziation durch Erhöhung der lonenkonzentration, durch Zugabe chaotroper Salze oder aussalzender Salze, durch Zugabe von Detergenzien, durch pH-Wert-Veränderung einzeln oder in Kombination erreicht werden.

Geeignete Dissoziationspuffer sind wässrige Pufferlösungen mit einer Molarität zwischen 0,01 bis 0,5 M, wie zum Beispiel Phosphatpuffer, Phosphat-Citrat-Puffer, Tris-HCl-Puffer, Tris-Acetat-Puffer, Carbonatpuffer, HCl-KCl-Puffer, HEPES-Puffer, Glycin-HCL-Puffer, MES-, MOPS-, PIPES-Puffer, Tricine-HCl-Puffer, und einem pH-Wert zwischen pH 2 und 10. Die Inkubation kann für 5 Minuten bis 3 Stunden erfolgen. In einer bevorzugten Ausführungsform ist der Dissoziationspuffer ein 0,05 M PBS oder ein 0,05 M Tris-HCl-Puffer, der chaotrope Salze enthält und der für 30 min im Reaktionsraum inkubiert wird.

Geeignete chaotrope Salze sind beispielsweise Harnstoff, Guadiniumhydrochlorid oder Natriumisothiocyanat in Konzentrationen von 0,1 M bis 6 M, bevorzugt 1 M bis 6 M.

Ebenso ist es möglich, den Analyten durch Zugabe von aussalzenden oder einsalzenden Salzen, wie beispielsweise 0,1 bis 6 M, bevorzugt 0,5 bis 5 M Natriumchlorid, vom Targetmolekül abzulösen.

In einer bevorzugten Ausführungsform erfolgt die chemische Dissoziation mit Dissoziationspuffern als Stufengradient oder als kontinuierlich ansteigender Gradient. Dazu können verschiedene konzentrierte Dissoziationspuffer nacheinander als Stufengradient mit Stufen von beispielsweise 0, 0,1, 0,5, 1, 2, 4 und 6 M eingesetzt werden oder durch einen Gradientenmischer als kontinuierlich ansteigender Gradient zwischen 0 und 6 M des jeweiligen Detergenz oder Salzes in den Reaktionsraum eingebracht werden. Je stärker der Analyt an das Targetmolekül bindet, umso höher ist die erforderliche Salz/Detergenzienkonzentration, um die Bindung des Analyten aufzulösen.

Ähnlich verhält es sich beim pH-Wert des Dissoziationspuffers, der einen großen Einfluss auf die Ionisierung funktioneller Gruppen und somit auf die Molekülladung insgesamt hat. Die Erniedrigung des pH-Wertes zwischen 2 und 7,4, oder die Erhöhung des pH-Wertes zwischen 7,4 und 12, können eingesetzt werden, um den Analyten vom Targetmolekül loszulösen.

Im Rahmen der chemischen Dissoziation ist es auch möglich, dem Dissoziationspuffer das spezifische oder ähnliche Targetmoleküle, in einer oder in verschiedenen Konzentrationen zuzusetzen, um die Bindung zwischen Targetmolekül und Analyten zu lösen.

Antikörper gegen Proteine können zum Beispiel durch Zugabe des spezifisch bindenden Peptides vom Targetmolekül abgelöst werden. Ein weiteres Beispiel sind Lektine, die an Oligosaccharide gebunden wurden und durch Zugabe der entsprechenden Monosaccharide abgelöst werden können.

In einer bevorzugten Ausführungsform erfolgt die Dissoziation physikalisch durch Temperaturerhöhung, isothermaler Inkubation, Schüttelinkubation oder Ultraschall. In einer besonders bevorzugten Ausführungsform der Erfindung erfolgt die physikalische Dissoziation durch Temperaturerhöhung des Reaktionsansatzes. Die thermische Dissoziation durch Erwärmung des Reaktionsansatzes hat den Vorteil, dass wahlweise und einfach die Stufen der Dissoziation auf den jeweiligen Test angepasst werden können.

Je stärker der Reaktionsansatz erwärmt wird, umso größer ist die Wahrscheinlichkeit, dass sich die Bindung zwischen Targetmolekül und Analyt löst und der Analyt in den Reaktionsraum diffundiert, was zu einer Verringerung der Fluoreszenzintensität im Bereich des immobilisierten Targetmoleküls führt. Bei welcher Temperatur des Reaktionsansatzes sich ein Analytmolekül vom Targetmolekül löst, hängt aber auch von der Affinität der Bindung ab. Je höher die Affinität der Bindung ist, umso höher ist auch die Temperatur/Energie, die benötigt wird, um den Analyten abzulösen.

In einer bevorzugten Ausführungsform der Erfindung erfolgt die Dissoziation durch Dissoziationspuffer als Stufengradient oder als kontinuierlich ansteigender Gradient und/oder durch Temperaturerhöhung als Temperaturstufengradient. Der Temperaturstufengradient kann verwendet werden, um den Schmelzpunkt des Analyten und des Referenzanalyten zu ermitteln.

In einer bevorzugten Ausführungsform der Erfindung wird ein Temperaturstufengradient genutzt, bei welchem in 0,1°C bis 5°C, bevorzugt 0,5°C bis 3°C, besonders bevorzugt 0,5°C bis 1°C, ganz besonders bevorzugt 0,5°C Schritten die Temperatur ermittelt wird, bei der die Fluoreszenzintensität am schnellsten abfällt. Dies stellt den Wendepunkt der Abschmelzkurve dar, für deren Generierung die Temperatur gegen die Fluoreszenzintensität graphisch aufgetragen wird. Dieser Wendepunkt wird auch als Schmelzpunkt bezeichnet.

Der Temperaturgradient kann zwischen 4 °C und 95°C, 20°C und 95°C, 25°C und 80°C, 30°C und 50°C zwischen 40°C und 65 °C und bevorzugt zwischen 37°C und 70°C erfolgen.

In einer besonders bevorzugten Ausführungsform des Verfahrens werden die Temperaturstufen auf die Schmelzpunkte des oder der Analyten beschränkt, sofern diese bekannt und zwischen verschiedenen Proben konstant sind. Es ist ebenso möglich, den Temperaturgradienten in verschiedenen Stufengraduierungen durchzuführen.

Die Erwärmung des Reaktionsansatzes kann für den Verfahrensschritt C) der Dissoziation besonders gut gesteuert werden und kann als Stufengradient oder homogener Gradient beispielsweise mit einem Thermocycler oder einem Heizblock realisiert werden, wobei der Einsatz des Thermocyclers bevorzugt ist. Der Thermocycler ermöglicht die ortsaufgelöste Detektion aller Fluoreszenzen vor, während und/oder nach der Dissoziation der Bindung zwischen Target- und Analytmolekülen im Reaktionsansatz.

In einer besonders bevorzugten Ausführungsform der Erfindung werden Thermocycler in Kombination mit einem Fluoreszenzmikroskop verwendet, mit derer es möglich ist, simultan zu einem physikalischen und/oder einem chemischen Gradienten Fluoreszenzmessungen bildgebend vom Reaktionsraum zu machen. Dabei kann die Grenzschicht der festen Phase zur Reaktionslösung automatisch fokussiert werden. Dazu werden bevorzugt Thermocycler eingesetzt, die in die Objektaufnahme eines Fluoreszenzmikroskopes passen und die Anwendung einer inversen Fluoreszenzoptik zulassen, in dem das Objektiv dicht genug an das Objekt herangeführt werden kann. Damit können optische Störeinflüsse vermieden werden. Existieren solche optischen Störeinflüsse nicht, kann die bildgebende Fluoreszenzmessung auch evers, das heißt durch die Reaktionslösung, insbesondere den Dissoziationspuffer hindurch, erfolgen. Optische Störeinflüsse können durch Autofluoreszenzen oder Absorptionen der Reaktionslösung, durch Verzerrungseffekte des Flüssigkeitsmeniskus der Reaktionslösung oder Kondensation von Reaktionslösung an der Objektivlinse des Messystems bedingt sein. Viele dieser Effekte können durch Verwendung von Abdeckungen des Reaktionsraumes oder Ölüberschichtung der Reaktionslösung vermieden werden.

In einer vorteilhaften Ausführungsform der Erfindung können chemische und physikalische Dissoziation kombiniert werden. Dadurch wird es möglich, den Intensitätsbereich der physikalischen Dissoziation zu verringern, um die Denaturierung von Target- bzw. Analytmolekülen oder des Reaktionsraumes, beispielsweise durch hohe Temperaturen zu vermeiden. Außerdem können niedrigaffine Bindungen dadurch aus der weiteren Analyse ausgeschlossen werden.

In einer vorteilhaften Ausführungsform der Erfindung wird bei einer Kombination der chemischen mit der physikalischen Dissoziation zunächst ein Stufengradient mit 6 M Harnstoff angewendet, um mit geringer und mittlerer Bindungsstärke gebundene Analyten vom jeweiligen Targetmolekül abzulösen. Durch nachfolgende schrittweise Temperaturerhöhung von beispielsweise 0,1 °C / 30 sec, 0,5 °C / 30 sec, 1 °C / 30 sec, 5°C / 30 sec, ist es möglich, ausschließlich die Ablösung der mit hoher Bindungsstärke an das jeweilige Targetmolekül gebundenen Analyten zu untersuchen. Die Dauer jeder Gradientenstufe kann zwischen 1 sec und 30 min liegen, bevorzugt zwischen 10 sec und 1 min und beträgt besonders bevorzugt weniger als 30 sec. Die Messungen können in einem Abstand von 1 sec bis 10 min, bevorzugt in einem Abstand von 5 sec bis 60 sec und besonders bevorzugt zwischen 10 und 30 sec erfolgen.

Da chemische und physikalische Dissoziation synergistisch wirken, ist es dadurch auch möglich die Temperatur zu verringern, die notwendig ist, um möglichst alle Analyten vom jeweiligen Targetmolekül abzulösen. Dadurch werden die Anforderungen, zum Beispiel an die thermische Toleranz des Reaktionsraumes, der Beads oder des Messsystems verringert.

Somit ermöglicht die feine Graduierung der Dissoziation der Bindung von Target- und Analytmolekülen die Aufzeichnung von Dissoziationskinetiken und zur Bestimmung der Schmelztemperatur.

### D) Messung der Nachweisfluoreszenz vor und während der Dissoziation

Erfindungsgemäß erfolgt die Messung der Nachweisfluoreszenz vor und während der Dissoziation ortsaufgelöst an mindestens zwei verschiedenen Messpunkten.

Die in Schritt C) beschriebene Dissoziation führt zu einer Verringerung der relativen Fluoreszenzmesswerte im Reaktionsansatz in Abhängigkeit der Stärke (Stringenz) der Dissoziationsbedingungen und der Bindungsstärke des Analyten an sein Targetmolekül sowie der Bindungsstärke der Nachweisfluoreszenz an Analyt und Referenzanalyt.

In einer bevorzugten Ausführungsform kann zur Messung der Nachweisfluoreszenz eine bildgebende Fluoreszenzmessung verwendet werden. Diese unterteilt sich in die Verfahrensschritte der automatischen Gerätesteuerung, der Bildaufnahme und der Bildauswertung.

Die Steuerung der Messtechnik erfolgt bevorzugt softwarebasiert, da eine genaue Abstimmung zwischen verschiedenen Einstellgrößen notwendig ist und je Objekt bei der Auswertung beispielsweise von Multiplex-Beadassays mehrere Bildaufnahmen erfolgen können. Mögliche Einstellgrößen sind beispielsweise die Steuerung der Objektpositionierung, des Autofokusses, der Objektbeleuchtung oder der Kamera. Im Falle der physikalischen Dissoziation kann der Thermocycler zur Temperatursteuerung und im Falle der chemischen Dissoziation ein Pipettierautomat zur Anwendung eines Salzstufengradienten eingesetzt werden.

Die Bildaufnahme von mikroskopischen Fluoreszenzbildern ist notwendig, um die vorstehend beschriebenen Multiplextests auswerten zu können. Die Bilder stellen selbst noch keine Messwerte dar, enthalten aber alle Bilddaten, um die Position aller Targetmolekülespezies und Referenzanalyten sicher zu bestimmen und die jeweilige Nachweisfluoreszenz zuordnen zu können.

Bevorzugt wird die Bildaufnahme durch ein inverses Fluoreszenzmikroskop oder daraus abgeleiteter Gerätesysteme durchgeführt. Das Fluoreszenzmikroskop kann einen motorisierten xy-Tisch bzw. z-Trieb, Objektivwechsler, Multibandanregungs-/Emissionsfilter inklusive der dazu passenden dichroitischen Spiegel, eine Mehrfach-LED-Beleuchtung, einen mikroskopoptischen, bevorzugt inversen Strahlengang oder eine CCD-Kamera aufweisen. Es können aber ebenso breitbandige Lichtquellen wie zum Beispiel Xenon-Lampen oder Quecksilber-Dampflampen in Kombination mit motorisierten Filterwechslern genutzt werden, um die Probe zu beleuchten. Ebenso ist es möglich Laser als Quelle des Anregungslichtes einzusetzen, wenn sehr hohe Anregungsenergien benötigt werden. Bevorzugt werden Messgeräte mit 3 bis 5 Messkanälen eingesetzt. Dadurch ist es möglich, mehr als eine Nachweisfluoreszenzmarkierung mit unterschiedlichen Fluoreszenzeigenschaften zu detektieren.

In einer Ausführungsvariante der Erfindung werden Objektivwechsler eingesetzt, um in derselben Messumgebung verschiedene Fragestellungen zu beantworten. Für eine Übersichtsaufnahme einer Mikrotestplatten-Kavität oder eines Objektträgers mit ortsaufgelösten Multiplex-Biochips oder mit Multiplex-Beadassays mit Spot- bzw. Beaddurchmessern größer 10 µm, sind Objektive mit einer Vergrößerung von 2-fach bis 10-fach ausreichend. Sind die Objekte kleiner, wie die fluoreszierenden Amplifikatspots nach isothermaler Amplifikation zur Einzelmoleküldetektion mit 1-5 µm Spotdurchmesser, kann mit dem Objektivwechsler einfach und schnell bei Bedarf ein 20-fach bis 40-fach Objektiv in den Strahlengang gebracht werden, falls sich die mikroskopische Auflösung als nicht ausreichend erweist.

In einer besonders bevorzugten Ausführungsform der Erfindung wird das Gerät zur Bildaufnahme mit einem Thermocycler kombiniert. Dieser kann beispielsweise ähnlich wie eine 96-Well-Mikrotestplatte in den Mikrotestplattenadapter des xy-Tisches montiert werden. Das ermöglicht die optimale Interaktion der hier beispielhaft genannten Prozesse Objektpositionierung, Bildaufnahme, Autofokus und Objekttemperierung.

Die Steuerung des Systems erfolgt bevorzugt durch eine modulare Software, die die genannten Hardwarekomponenten ansteuern kann, um Fluoreszenzbilder vor und während Dissoziation aufzunehmen. Daraus können dann durch digitale Bildauswertung Messwerte erzeugt werden, die ausgewertet werden können, um das Vorhandensein eines Analyten feststellen und/oder Bindungsstärken der Bindung des Analyten an sein jeweiliges Targetmolekül erfassen zu können.

Bei der Verwendung von ortskodierten Biochips, bei denen die Targetmoleküle und/oder Referenzanalyten in zweidimensionaler Anordnung auf die feste Phase gedruckt werden können, resultiert beispielsweise ein definiertes Punkteraster, wobei jeder Punkt abgesehen von Replikaten einer Targetmolekülspezies entspricht. Die Bildauswertesoftware kann das Punkteraster und die darin positiv reagierenden Punkte erkennen und den immobilisierten Biomolekülen zuordnen. Die Positionen der positiven Punkte in Form von Microspots mit einem Durchmesser von 1 µm bis 1.000 µm können dann bei der nachfolgenden Dissoziation anhand der Ortskoordinaten der Punkte wiedergefunden werden, auch wenn mit zunehmender Dissoziation keine Nachweisfluoreszenz mehr vorhanden ist. Solche Systeme können mit einer Fluoreszenzfarbe, das heißt, einem Fluoreszenzmesskanal, ausgewertet werden, wobei in der Regel zwei oder mehr Fluoreszenzfarben angewendet werden können.

Im Falle der Einzelmoleküldetektion findet keine schrittweise Verringerung der Fluoreszenz der Spots statt. Da sich die Analyt-Moleküle einzeln vom gebundenen Targetmolekül ablösen, verschwinden die Spots bei Erreichen der Dissoziationsbedingungen sofort vollständig. Es gibt nur die Zustände "Spot sichtbar" bzw. "Spot nicht mehr sichtbar", weshalb das Detektionsprinzip als "digital" bezeichnet wird. Die Anzahl der Spots, die softwarebasiert je Dissoziationsstufe aus den Fluoreszenzaufnahmen ausgezählt wird, korreliert mit der Affinität des Analyten. Je mehr Analyten je Dissoziationsstufe gezählt werden, umso höher ist die Affinität des Analyten und entsprechender Subpopulationen von Analytmolekülen. Verschwinden alle Analytmoleküle in einer oder wenigen Dissoziationsstufen, so kann es sich hier um eine monoklonale Spezifität des Analyten handeln. Es ist zu beachten, dass Antikörper unterschiedlichen Anzahlen von Paratopen besitzen und die Stärke der Bindung auch davon abhängt, dass ein Antikörper mit einer oder mehreren Bindungsstellen an Targetmolekülen binden kann. Hier kann beispielsweise ein Pepsinverdau vor Bindung der Nachweisfluoreszenzmarkierung durchgeführt werden, um z.B. aus bivalenten IgG-Antikörpern monovalente IgG-Fab-Fragmente, gebunden am Targetmolekül, zu erzeugen. Das setzt voraus, dass das Targetmolekül bevorzugt nicht oder nicht so stark von Pepsin gespalten werden kann. Der Pepsinverdau kann alternativ vor Inkubation der Probenlösung im Reaktionspuffer erfolgen, wobei zur Beendung des Pepsin-Verdaus dieses durch z.B. Zugabe von Proteaseinhibitoren oder durch Temperaturerhöung inaktiviert wird. Diese Ausführungsform ist besonders dann geeignet, wenn die Targetmoleküle Proteine sind, die durch Pepsin abgebaut werden können.

Bei der Verwendung von Beadassays können die Beads zufällig verteilt am Boden des Reaktionsraumes vorliegen. Durch die Beadkodierung über die Beadgröße und oder die Fluoreszenzseigenschaften, werden 1 bis 3 Fluoreszenkanäle benötigt, um die Zugehörigkeit aller Beads zur jeweiligen Beadpopulation bestimmen zu können. Da die Beads bevorzugt permanent am Boden des Reaktionsraumes immobilisiert vorliegen können, ist es auf diese Weise möglich, die Position der Beads und somit der zugehörigen Targetmolekülespezies und Referenzanalyten einfach bestimmen zu können, selbst, wenn die Nachweisfluoreszenz gemeinsam mit dem Analyten während der Dissoziation abgelöst wird.

In einer bevorzugten Ausführungsform der Erfindung werden Beadassays zur Messung der Nachweisfluoreszenz verwendet. Dies ermöglicht in vorteilhafterweise, die Nachweisfluoreszenz über eine Beadkodierungsfluoreszenz zu referenzieren, um Größenunterschiede zwischen den Beads und daraus resultierende Messfehler besser ausgleichen zu können.

In einer Ausführungsform der Erfindung können pro Beadpopulation, 1 bis 10.000 Beads pro Reaktionsansatz, bevorzugt 10 bis 100 Beads und besonders bevorzugt 20 bis 50 Beads gemessen werden. Dadurch kann die Streuung der Messwerte für die Nachweisfluoreszenz je Beadpopulation erheblich reduziert werden. Die relativen Fluoreszenzmesswerte der Nachweisfluoreszenz können damit direkt oder verrechnet als Ratio aus Cut-off, dem Positivschwellwert, und dem Messwert für die jeweilige Targetmolekülspezies ausgegeben werden.

In einer weiteren Ausführungsform der Erfindung kann sich die Spotgröße nach isothermaler Amplifikation, die an verschiedene Analytmoleküle einer Analytspezies gebunden ist, unterscheiden. Bei der Dissoziation wirken deshalb unterschiedliche Kräfte an den Analyten, was die Dissoziation vereinfachen oder erschweren kann. Deshalb kann ist es sinnvoll, bei der Berechnung der Dissoziation von Einzelmolekülen nicht nur die Stufe des Dissoziationsgradienten zu berücksichtigen, sondern auch die Spotgröße oder die Spothelligkeit, um Fehlermöglichkeiten zu reduzieren. Die Spotgröße ergibt sich bei Aufnahme der Fluoreszenzbilder mit einer CCD-Kamera aus der Zahl der Pixel pro Spot und die Spothelligkeit aus der Summe der Pixelhelligkeiten aller Pixel des Spots. Die Größe des Spots wird bevorzugt als Quotient aus Spothelligkeit und Pixelanzahl berechnet und wird als mittlere Pixelhelligkeit bezeichnet. Alternativ ist es möglich, als Ausdruck für die Spotgröße nur die Pixelzahl oder nur die Spothelligkeit zu verwenden. Welches Verfahren besser geeignet ist, sollte empirisch für jedes Testsystem ermittelt werden. Als Analyten sollten standardisierte Reagenzien, wie zum Beispiel monoklonale oder rekombinante Antikörper eingesetzt werden. Die Spotgröße des betrachteten Spots wird auf die durchschnittliche Spotgröße aller Spots des Fluoreszenzbildes bezogen, um die Abweichungen jedes Spots vom Mittelwert als Maß für die wirkenden Kräfte zu nutzen. Abschließend ist der Einfluss der Spotgröße für jedes Testsystem zu ermitteln, indem die mathematische Funktion ausgewählt wird, die in Bezug auf standardisierte Analyten die geringsten Streuungen des Mittelwertes aller ermittelten Dissoziationsstufen liefert. Ein Beispiel einer Funktion, die genutzt werden kann, legt einen linearen Zusammenhang zwischen Spotgröße sowie der Genauigkeit der Dissoziation zugrunde, so dass jede Dissoziationsstufe mit der prozentualen Abweichung der Spotgröße vom Mittelwert aller Spotgrößen mit der Dissoziationsstufe multipliziert wird. Als Beispiel wird ein Spot betrachtet, der um 10% vom Mittelwert aller Spots nach oben abweicht. Wenn dieser Spot bei der Gradientenstufe 55°C dissoziiert, würde sich nach dem Rechenmodell eine Erhöhung um 10 %, das heißt um 5,5°C ergeben, so dass die rechnerisch korrigierte Gradientenstufe 60,5 °C betragen würde. Nach Korrektur aller Gradientenstufen wird der Mittelwert aller Gradientenstufen gebildet und mit dem Mittelwert aller Gradientenstufen aller Spots vor Korrektur verglichen. Ist die Streuung des Mittelwertes geringer geworden, wird die verwendete Funktion akzeptiert. Da die Auswirkung der Spotgröße in verschiedenen Testsystemen auf die Dissoziation der Analyten von den Targetmolekülen ganz unterschiedlich sein kann, können andere mathematische Funktionen deutlich besser geeignet sein als die zuvor genannte. Deshalb ist das zuvor genannte Verfahren für beliebig weitere mathematische Funktionen zu wiederholen, bis die optimale Korrekturfunktion gefunden wurde. In bevorzugten Testsystemen, bei denen die Spotgröße keinen Einfluss auf den ermittelten Dissoziationsgradienten hat, kann auf eine Korrektur verzichtet werden. Das ist dann gegeben, wenn die Streuung des Dissoziationsgradienten unter 50 %, unter 20 % und bevorzugt unter 10 % liegt, je nachdem was als Intra-Assay-Variation für die konkrete Testfragestellung akzeptabel ist.

Ein weitere Ausführungsform kann die Zuordnung der Targetmoleküle in histologischen oder cytologischen Präparaten sein, die oft auf spezifische Zell- oder Gewebestrukturen beschränkt sein kann. Hier kann zur Auswertung der Nachweisfluoreszenzmarkierung zusätzlich eine Mustererkennung für Zell- und Gewebestrukturen benötigt werden. In der Regel kann ein Fluoreszenkanal eingesetzt werden, um beispielsweise die Zellkerne als Orientierungsstrukturen für die Lokalisierung von Zellen mit DAPI (4',6-Diamidin-2-phenylindol) anfärben und messen zu können. Ein oder mehrere Fluoreszenzmesskanäle können für die Aufzeichnung der Nachweisfluoreszenz genutzt werden.

### E) Bestimmung der Bindungsstärke des Analyten

In einer Ausführungsform der Erfindung können die relativen Fluoreszenzmesswerte graphisch gegen die Stringenz der Dissoziationsbedingungen aufgetragen werden, um visuell die Bindungsstärke zu beurteilen.

Verlaufen die Kurven der relativen Fluoreszenzmesswerte von Analyt und Referenzanalyt parallel, dann ist dies ein Hinweis darauf, dass sich ausschließlich die Bindung zwischen Nachweisfluoreszenzmarkierung und Analyt löst und nicht zwischen Analyt und Targetmolekül. In diesem Falle kann angenommen werden, dass der Analyt eine höhere Bindungsstärke zum Targetmolekül als zur Nachweisfluoreszenzmarkierung besitzt. Eine Charakterisierung einer sehr starken Bindung von Analyt an sein Targetmolekül, kann dann nicht weiter durchgeführt werden, ohne die Bindung von Nachweisfluoreszenzmarkierung an den Analyten beispielsweise durch kovalente Bindung zu erhöhen bzw. die Valenz der Bindung des Analyten von z.B. bivalent auf monovalent durch enzymatischen Verdau zu verringern.

Fällt der relative Fluoreszenzmesswert eines Analyten hingegen schnell ab und der relative Fluoreszenzwert des Referenzanalyten bleibt auf hohem Niveau konstant, dann kann von einer Dissoziation ausschließlich zwischen Targetmolekül und Analyten ausgegangen werden. Meist verringern sich die relativen Fluoreszenzmesswerte von Analyt und Referenzanalyt in einer Weise, die zwischen den Beiden zuvor geschilderten Fällen liegt.

Durch das erfindungsgemäße Verfahren ist es jedoch möglich, den relativen Fluoreszenzmesswert der Analyten einer jeden Dissoziationsstufe, bezogen auf den Referenzanalyten, zu korrigieren. In einer bevorzugten Ausführungsform des Verfahrens kann die prozentuale Verringerung des relativen Fluoreszenmesswertes des Referenzanalyten von dem des Analyten für die jeweilige Dissoziationsstufe abgezogen werden.

In einer alternativen Ausführungsform des Verfahrens kann der Quotient der relativen Fluoreszenzmesswerte von Analyt und Referenzanalyt gebildet werden und entsprechend graphisch dargestellt werden.

Es ist ebenfalls möglich die Referenzfluoreszenz separat, zum Beispiel für jede Testcharge oder als Parallelbestimmung innerhalb einer Probenserie, zu bestimmen.

In einer bevorzugten Ausführungsform der Erfindung erfolgt ein systematischer softwarebasierter Vergleich der Bindungsstärken, wobei die Messung der Nachweisfluoreszenz vor und während der Dissoziation ortsaufgelöst an mindestens zwei verschiedenen Messpunkten erfolgen kann.

Dazu kann der Messwert einer bestimmten Dissoziationsstufe für alle Proben ausgegeben werden. Bei physikalischen oder chemischen Stufengradienten kann auch der Wendepunkt mittels der zweiten Ableitung aus dem Datensatz eines Analyten für alle aufgezeichneten Dissoziationsstufen berechnet werden.

Alternativ kann die Dissoziationsstufe des Stufengradienten der halbmaximalen relativen Fluoreszenzintensität der Analytfluoreszenz als Maß für die Bindungsstärke angegeben werden.

In einer bevorzugten Ausführung der Erfindung kann die Temperaturstufe angegeben werden, bei der die relative Fluoreszenzintensität 50% des Maximalwertes beträgt. Diese wird als Schmelztemperatur bezeichnet. Die Schmelztemperatur des Analyten kann somit mit sehr hoher Reproduzierbarkeit bestimmt werden, so dass Veränderungen des Analyten, die sich auf die Bindungsstärke und somit auf die Schmelztemperatur auswirken, sehr genau erkannt werden können. Bevorzugt erfolgt die Bestimmung der Schmelztemperatur mit einem Thermocycler. Dies hat den Vorteil hat, dass die Schwankungen der Schmelztemperatur sehr gering sind.

In einer Ausführungsform der Erfindung kann die Intensität der Nachweisfluoreszenzmarkierung für jeden Messpunkt als Funktion der Zeit bei isothermaler Reaktionsführung oder als Funktion der Temperatur und/oder der Salzkonzentration bei Anwendung eines Stufengradienten aufgezeichnet werden und der Zeitwert oder der Temperaturwert und/oder Salzkonzentrationswert des Wendepunktes oder der halbmaximalen Fluoreszenzintensität oder eines anderen definierten Punktes, wie beispielsweise der zweifache oder dreifache Wert des Analyt-spezifischen Cut-offs oder der statistisch signifikant veränderte Anstieg des Graphen der resultierenden Messwertreihen, als Maß für die Avidität des Analyten ermittelt werden.

In einer weiteren Ausführungsform der Erfindung kann zur Berechnung des Schmelzpunktes des Analyten die Fluoreszenzintensität, die Temperatur oder die Zeit im Wendepunkt oder einem anderen definierten Punkt der aufgezeichneten Fluoreszenzsignale eines Analyten von der Fluoreszenzintensität, der Temperatur oder der Zeit im Wendepunkt oder einem anderen entsprechenden Punkt der aufgezeichneten Fluoreszenzsignale der Referenzfluoreszenz der an ein Referenzanalyten gebundenen Nachweisfluoreszenzmarkierung subtrahiert werden.

Bei Verfügbarkeit von standardisierten Proben mit bekannter Bindungsstärke ist es somit möglich, die Bindungsstärke des Analyten zu kalibrieren. Standardisierte Proben sind beispielsweise monoklonale oder rekombinante Antikörper, deren Bindungsstärke vorab mit Vergleichsverfahren wie der Surface Plasmon Resonance bestimmt wurden. Der Vorteil standardisierter kalibrierter Verfahren besteht auch darin, dass nicht in jedem Testansatz der Referenzanalyt mitbestimmt werden muss. Standardisierte Proben können im parallelen Ansatz in der Testserie als externer Kalibrator mitbestimmt werden. Alternativ können monoklonale oder rekombinante Antikörper, deren Dissoziationsverhalten bekannt und auch unter dem Einfluss der Probenmatrix stabil ist, der Probe als interner Kalibrator zugesetzt werden. Abweichungen der Dissoziationskinetiken oder des Schmelzpunktes dieses internen Kalibrators sind dann auf Probenantikörper zurückzuführen. Eine Verringerung des Schmelzpunktes deutet auf Antikörper mit niedrigerer Affinität als der interne Kalibrator-Antikörper in der Probe hin. Erhöht sich der Schmelzpunkt, befinden sich folglich höheraffine Antikörper in der Probe. Dieses Prinzip kann vom Fachmann auch auf andere Analyten-Klassen übertragen werden. Die Zugabe von internen Kalibratoren wird bevorzugt bei der Einzelmoleküldetektion genutzt.

Bei allen Berechnungsverfahren ist bei Bedarf der relative Fluoreszenzwert des Analyten durch Referenzierung auf den Referenzanalyten entsprechend der beschriebenen Verfahren zu korrigieren.

Das gilt ebenso bei der Einzelmoleküldissoziation nach isothermaler Amplifikation des Labels am Analytbinder zur Komplettierung der Nachweisfluoreszenzmarkierung. Auch hierbei muss sichergestellt werden, dass bevorzugt die Bindung des Analyten an das Targetmolekül und nicht an den Analytbinder in Schritt C) dissoziiert.

### Ausführungsbeispiele und Figurenbeschreibung

Die Beispiele dienen ausschließlich der Illustration und beschränken die Erfindung nicht.

### Figuren

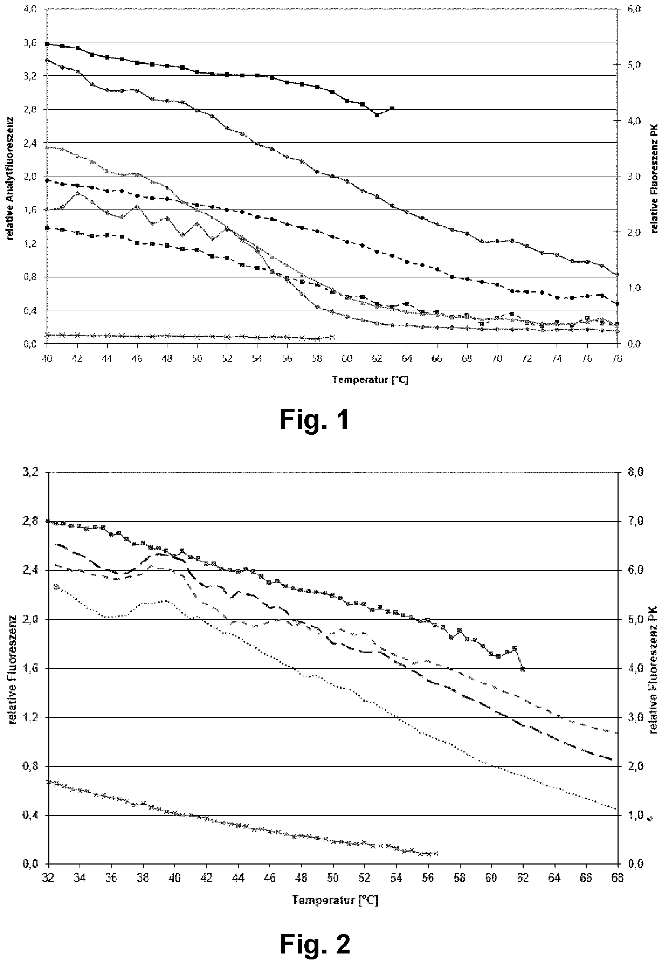

In **Figur 1** (Anti-SARS-CoV-2-RBD-Dissoziationskurven) ist eine bevorzugte Ausführungsform der vorliegenden Erfindung dargestellt. Fig. 1 zeigt die Dissoziationskurven gebundenen IgG-Antikörper an die SARS-Co2-Rezeptorbindungsdomäne für fünf Patientenseren im Antikörper-Beadassay. Nicht valide Messpunkte für die NK und die PK bei höheren Temperaturen wurden aus der Messreihe entfernt. Die Dissoziationskurven zeigen einen linearen bis sigmoidalen Verlauf und die Schmelzpunkte liegen zwischen 54°C und 62°C.
   **Fig. 1****:** NK: Negativkontrolle (-X-); PK: Positivkontrolle (-▪-), Serum 3 (--■--), Serum 4 (- - • - -), Serum 5 (-◆-), Serum 6 (-▲-), Serum 7 (-•-)
In **Figur 2** (Anti-CCP-IgG-Dissoziationskurven Serum 3) ist eine bevorzugte Ausführungsform der vorliegenden Erfindung dargestellt. Fig. 2 zeigt die Dissoziationskurven aus einem Patientenserum (Serum 3) gebundener IgG-Antikörper an verschiedenen citrullinierten Peptiden im Antikörper-Beadassay. Im Temperaturgradienten und kombinierten Harnstoff-Temperaturgradienten verringern sich die relativen Analytfluoreszenzen. Fig. 2 (Anti-CCP-IgG-Dissoziationskurven für citrulliniertes Filaggrin für Serum 3) zeigt Daten eines ausgewählten Peptides (für citrulliniertes Filaggrin, **SEQ ID NO. 5**) im Harnstoff-Temperaturgradienten für Serum 3. Mit zunehmender Harnstoffkonzentration bzw. Temperatur verringern sich die relativen Analytfluoreszenzen und die Schmelztemperaturen der gebundenen Anti-citrullinierter Filaggrin-IgG-Antikörper (Anti-Cit-Fil).
   Nicht valide Messerwerte der NK und der PK bei höheren Temperaturen wurden aus den Messreihen entfernt. NK: Negativkontrolle; PK: Positivkontrolle.
   **Fig. 2****:** NK 0M Harnstoff (-X-); PK 0M Harnstoff (-▪-), Filaggrin Standard (-- -), Filaggrin 3 M Harnstoff (- - - -), Filaggrin 6 M Harnstoff (......)

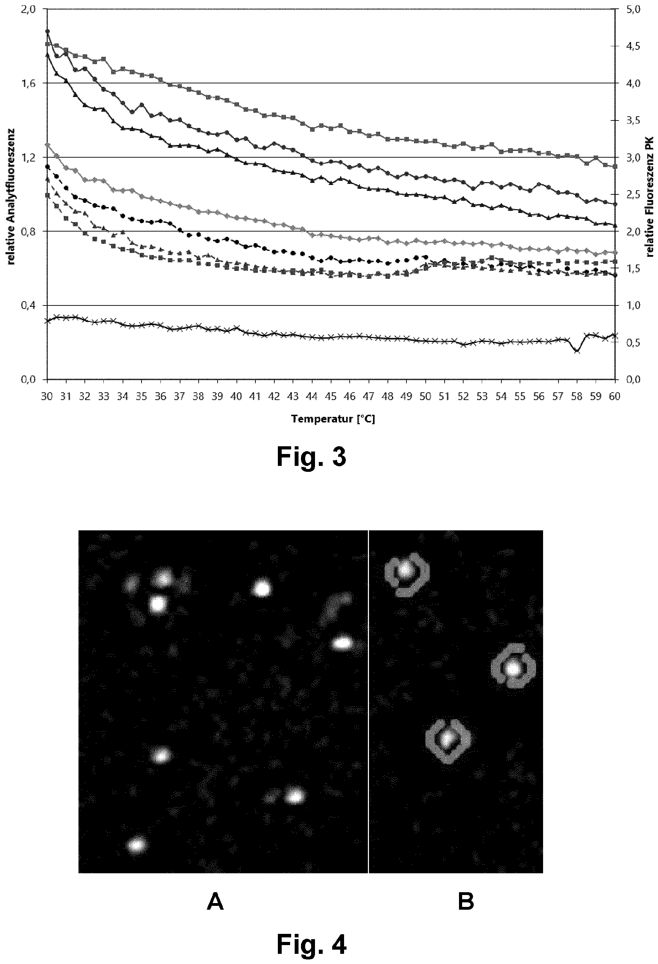

In **Figur 3** (Anti-Ro60-IgG Dissoziationskurven) ist eine bevorzugte Ausführungsform der vorliegenden Erfindung dargestellt. Fig. 3 zeigt die Dissoziationskurven an das Autoantigen Ro60 gebundener IgG-Antikörper für 1 Patientenserum im Antikörper-Beadassay. Die Dissoziationskurven bei variierenden NaCL-Konzentrationen im Dissoziationspuffer zeigen einen nichtlinearen Abfall der relativen Analytfluoreszenzen und die Schmelzpunkte liegen zwischen 44°C und 32°C.
   **Fig. 3****:** NK: Negativkontrolle (-X-); PK: Positivkontrolle (-▪-), PBS-Puffer, 0,15 M (-•-), 1 M NaCl (-▲-), 2 M NaCl (-◆-), 3 M NaCl (- -•- -), 4 M NaCl (- -▲- -), 5 M NaCl (- - ▪ - -)
In **Figur 4** ist eine bevorzugte Ausführungsform der vorliegenden Erfindung dargestellt. Fig. 4 zeigt fluoreszierende Spots gebundener IgG-Antikörper aus einem Patientenserum gebundener IgG-Antikörper nach isothermaler Amplifkation einer fluoreszierenden Markierung des gebundenen Anti-human-IgG (Nachweisfluoreszenzmarkierung) und Einzelmoleküldetektion (a). Durch die Software Microspot-Analyzer werden valide Spots rot markiert, dann die Daten extrahiert und für die nachfolgende Auswertung bereitgestellt.
   Fig. 4: A) Amplifikat vor Softwareauswertung, B) Nach Softwareauswertung mit dem "Spot-Analyser" werden alle erkannten Amplifikat-Spots automatisch markiert und gezählt.

### Beispiele

### Beispiel 1:

### Nachweis von humanen Antikörpern gegen die Rezeptor-Bindungsdomäne (RBD) des SARS-Cov-2 Viruses und Bestimmung der Schmelzpunkte der gebundenen Antikörper

Zur Herstellung der Beadassays werden die RBD (Hytest), humanes Serum Albumin (Sigma) und humanes IgG (Sigma) mittels Carbodiimid-Kopplung an verschiedene Populationen Carboxy-modifizierter, dual-colour-kodierter Beads (PolyAn) gekoppelt. Die gekoppelten Beads werden in PBS mit 10% Glycerin zu einer Beadsuspension vermischt. Aliquots dieser Suspension werden jeweils in eine polylysinbeschichtete Kavität (Diapops-Verfahren, Nunc) von 8er-Modulen (NukleoLink^{®}, ThermoFisher Scientific) gegeben. Nach 20-minütiger Sedimentation und anschließendem mehrmaligem Waschen mit Aqua dest. befinden sich ca. 50 Beads jeder Population je Kavität. Die 8er-Module werden bis zur Testdurchführung bei 4°C dunkel gelagert.

Zur Testdurchführung werden Patientenseren 1:41 in PBS-T verdünnt und jeweils 205 µL in eine der vorbereiteten Beadassay-Kavitäten pipettiert und für 1,5 h inkubiert. Nach dreimaligem Waschen mittels PBS-T wird Anti-human-lgG-Atto647N-Konjugat (Nachweisfluoreszenzmarkierung) 1:10 verdünnt in PBS-T in die Kavitäten pipettiert und für 1,5 h inkubiert. Nach dreimaligem Waschen werden 50 µL PBS-T in die Kavitäten gegeben. Danach erfolgt die ortsaufgelöste Fluoreszenzmessung der Kavitäten im Attomol^{®} Caldeidoscan 300 (CS300, Firma Attomol) bei Raumtemperatur, in den der Thermocycler Attomol^{®} Caldeidoamp 100 (CA100, Firma Attomol) integriert wurde. Die Steuerung des Messgerätes und des Thermocyclers sowie die Aufnahme und Verarbeitung der Bilddaten bis hin zur Ausgabe der relativen Fluoreszenzmesswerte erfolgt entsprechend Herstellerangaben mit Hilfe der Steuer- und Analysesoftware Attomol^{®} Caleidopro 1.0 (CP1.0, Fa. Attomol). Danach wird die Temperatur in 0,5°C-Schritten von 35 °C bis 70 °C erhöht. Innerhalb jeder Temperaturstufe erfolgt eine Bildaufnahme sowie Auswertung der Bilddaten. Nach dem Abschluss der aller Messschritte wurden die Daten exportiert und in EXCEL graphisch aufbereitet. Die Ergebnisse sind in Fig. 1 dargestellt und erläutert. Fig. 1 zeigt die Dissoziationskurven gebundener IgG-Antikörper aus verschiedenen Patientenseren. Der Analyt, humane Anti-RBD-Antikörper aus Patientenserum bindet im Gegensatz zur Negativkontrolle stark an das jeweilige Antigen. Das trifft ebenso für die Bindung der Nachweisfluoreszenzmarkierung an die Positivkontrolle zu, die eine maximale relative Fluoreszenz von 5,4 erreicht. Im weiteren Verlauf der Messungen verringern sich die Bindungen der verschiedenen Serumantikörper an die Antigene mit zunehmender Temperatur unterschiedlich stark. Die Bindung an den Referenzanalyten (Positivkontrolle) verringert sich um etwa 20 %. Die der Patientenseren verringert sich bei 60°C um etwa 85 % (Serum 5). Die Schmelztemperaturen für Anti-SARS-Cov-2-IgG-Antikörper wurden graphisch bestimmt und liegen zwischen ca. 54 °C (Serum 6) bzw. 62 °C (Serum 7). Die Kurvenform variiert von sigmopidal bis nahezu linear und lässt auf unterschiedliche Dissoziationskinetiken der Serumantikörper gegen die SARS-Co2-RBD schließen.

Da die Beads für die Positivkontrolle und Negativkontrolle nicht ausreichend thermostabil sind, wurden die Messwerte ab 64°C für die Positivkontrolle und ab 60°C für die Negativkontrolle als nicht valide eingeschätzt und aus der Messreihe entfernt. Hier ist zur Vereinfachung die Positivkontrolle und die Negativkontrolle nur für den Reaktionsansatz von Serum 3 gezeigt. Die Reaktionsansätze für die übrigen Seren enthalten ebenfalls jeweils beide Kontrollen, die ähnlich wie hier gezeigt reagieren.

### Beispiel 2:

### Nachweis von humanen Antikörpern gegen citrullinierte Peptide und Bestimmung der Schmelzpunkte der gebundenen Antikörper

Zur Herstellung der Beadassays werden die citrullinierten Peptide (Firma Attomol, REF1271), humanes Serum Albumin (Sigma) und humanes IgG (Sigma) mittels Carbodiimid-Kopplung an verschiedene Populationen Carboxy-modifizierter, dual-colour-kodierter Beads (PolyAn) gekoppelt. Die gekoppelten Beads werden in PBS mit 10% Glycerin zu einer Beadsuspension vermischt. Aliquots dieser Suspension werden jeweils in eine polylysinbeschichtete Kavität (Diapops-Verfahren, Nunc) von 8er-Modulen (NukleoLink^{®}, ThermoFisher Scientific) gegeben. Nach 20-minütiger Sedimentation und anschließendem mehrmaligem Waschen mit Aqua dest. befinden sich ca. 50 Beads jeder Population je Kavität. Die 8er-Module werden bis zur Testdurchführung bei 4°C dunkel gelagert.

Zur Testdurchführung wird ein antikörperpositives Patientenserum 1:40 in PBS-T verdünnt und jeweils 200 µL in eine der vorbereiteten Beadassay-Kavitäten pipettiert und für 1,5 h inkubiert. Nach dreimaligem Waschen mittels PBS-T wird Anti-human-lgG-Atto647N-Konjugat (Nachweisfluoreszenzmarkierung) 1:10 verdünnt in PBS-T in die Kavitäten pipettiert und für 1,5 h inkubiert. Nach dreimaligem Waschen werden 50 µL PBS-T, der jeweils verschiedene Konzentrationen Harnstoff (0 M bis 6 M) enthält, in die Kavitäten gegeben. Danach erfolgt die ortsaufgelöste Fluoreszenzmessung der Kavitäten im CS300 bei Raumtemperatur, in den der Thermocycler CA100 integriert wurde. Die Steuerung des Messgerätes und des Thermocyclers sowie die Aufnahme und Verarbeitung der Bilddaten bis hin zur Ausgabe der relativen Fluoreszenzmesswerte erfolgt entsprechend Herstellerangaben mit Hilfe der Steuer- und Analysesoftware CP1.0. Danach wird die Temperatur in 0,5°C-Schritten von 35 °C bis 68 °C erhöht. Innerhalb jeder Temperaturstufe erfolgt eine Bildaufnahme sowie Auswertung der Bilddaten. Nach dem Abschluss aller Messschritte wurden die Daten exportiert und in EXCEL rechnerisch und. graphisch aufbereitet. Die Ergebnisse sind in Fig. 2 dargestellt und erläutert. Fig. 2 zeigt die Dissoziationskurven der aus einem Patientenserum gebundenen IgG-Antikörper.

Fig. 2: Gezeigt wird die Dissoziation vom citrullinierten Filaggrin-Antigen (bei verschiedenen Harnstoffkonzentrationen: 0 M, 3 M, 6 M) sowie vom Referenzanalyt und der Negativkontrolle (jeweils ohne Harnstoff) für Serum 3. Humane Anti-citrulliniertes Filaggrin-IgG-Antikörper binden im Gegensatz zur Negativkontrolle stark an das citrullinierte Filaggrin-Antigen. Im weiteren Verlauf der Messungen verringern sich die Bindungen an das Antigen in Abhängigkeit der Harnstoffkonzentration und der Temperatur unterschiedlich stark. Die Bindung an den Referenzanalyten, d.h. der Positivkontrolle PK, verringert sich ohne Harnstoffzusatz bei 60°C auf etwa 65% der ursprünglichen Signalhöhe und die an citrulliniertes Filaggrin-Antigen auf etwa 56%, was auf einen relativ geringen Unterschied im Dissoziationsverhalten des Referenzanalyten und des Analyten hindeutet. Die Dissoziation der Antikörper nimmt mit steigender Harnstoffkonzentration zu. Dennoch ist festzustellen, dass die Dissoziationskurven für die verschiedenen Harnstoffkonzentrationen fast parallel verlaufen. Diese beiden Befunde legen nahe, dass Serum 3 hochavide Anti-citrulliniertes Filaggrin-Antikörper enthält. Die graphisch ermittelte Schmelztemperatur liegt für die verschiedenen Harnstoffkonzentrationen bei: 58 °C (0 M), 64 °C (3M), 54 °C (6 M). Bemerkenswert ist, dass die Dissoziationskurve für 0 M Harnstoff im höheren Temperaturbereich schneller abfällt als bei 3 M Harnstoff und dadurch die Schmelztemperatur bei 0 M Harnstoff höher ist als bei 3 M Harnstoff. Es fällt weiterhin auf, dass die Dissoziationskurven im mittleren Temperaturbereich untypisch unruhig verlaufen. Beide Effekte sprechen für eine Epitopinstabilität des citrullinierten Filaggrin-Peptid-Antigens.

### Beispiel 3:

### Nachweis von Antikörpern gegen extrahierbare nukleäre Antigene (ENA) und Bestimmung der Bindungsstärke mit Hilfe chemischer (NaCi-Stufengradient) und physikalischer Dissoziation (Temperaturstufengradient)

Zur Herstellung der Beadassays werden verschiedene ENA (Attomol, REF1111), humanes Serum Albumin (Sigma) und humanes IgG (Sigma) mittels Carbodiimid-Kopplung an verschiedene Populationen Carboxy-modifizierter, dual-colour-kodierter Beads (PolyAn) gekoppelt. Durch drei Waschschritte werden nicht an Beads gebundene Bestandteile der jeweiligen Suspension entfernt. Aliquots jeder Suspension werden gemischt und in polylysinbeschichtete Kavitäten (Diapops-Verfahren, Nunc) von 8er-Modulen (Nukleolink, TermoFisher) gegeben, so dass ca. 50 Beads jeder Population je Kavität nach Durchführung aller Waschschritte der nachfolgenden Testdurchführung vorhanden sind. Nach Sedimentation der Beads werden die 8er-Module bis zur Testdurchführung bei 4°C dunkel gelagert.

Zur Testdurchführung werden Patientenseren 1:100 in PBS-T verdünnt und jeweils 50 µL in eine der vorbereiteten Beadassay-Kavitäten pipettiert und für 1 h inkubiert. Nach dreimaligem Waschen mittels PBS-T wird Anti-human-lgG-Atto647N-Konjugat (Nachweisfluoreszenzmarkierung) verdünnt in PBS-T in die Kavitäten pipettiert und für 1 h inkubiert. Nach dreimaligem Waschen werden 50 µL PBS-T in die Kavitäten gegeben. Danach erfolgt die ortsaufgelöste Fluoreszenzmessung der Kavitäten im CS300 bei Raumtemperatur, in den der Thermocycler CA100 integriert wurde. Die Steuerung des Messgerätes und des Thermocyclers sowie die Aufnahme und Verarbeitung der Bilddaten bis hin zur Ausgabe der relativen Fluoreszenzmesswerte erfolgt entsprechend Herstellerangaben mit Hilfe der Steuer- und Analysesoftware CP1.0. Danach wird NaCL in PBS-T in der jeweilig gewünschten Konzentration dazugegeben und die Messung bei Raumtemperatur wiederholt. Danach wird die Temperatur in 0,5°C-Schritten von 35 °C bis 60 °C erhöht. Innerhalb jeder Temperaturstufe erfolgt eine Bildaufnahme sowie Auswertung der Bilddaten. Nach dem Abschluss aller Messschritte wurden die Daten exportiert und in EXCEL graphisch aufbereitet. Die Ergebnisse sind in Fig. 3 nur für die dissoziationskurven von Anti-Ro60-IgG-Antikörper dargestellt und erläutert. Fig. 3 zeigt die Dissoziationskurven aus einem Patientenserum gebundener IgG-Antikörper im Temperaturgradienten und kombinierten NaCL-Temperaturgradienten.

Es werden nur die Ergebnisse mit dem ENA-Beadassay REF1111 für Ro60 dargestellt. Das Antigen Ro60 bindet stark Anti-Ro60-Antikörper aus der Probe, die ihrerseits die Nachweisfluoreszenzmarkierung mit einem maximalen relativen Analytfluoreszenzwert von 1,9 binden. Der Referenzanalyt humanes IgG bindet die Nachweisfluoreszenzmarkierung im Gegensatz zur Negativkontrolle sehr stark mit einem relativen Fluoreszenzmesswert PK von 4,5. Im weiteren Verlauf der Messungen verringern sich die Bindungen der Anti-Ro60-Antikörper an das Antigen Ro60 unterschiedlich stark in Abhängigkeit der NaCl-Konzentration. Die Bindung an den Referenzanalyten verringert sich ebenfalls. Die Dissoziationskurven von Ro60 und dem Referenzanalyten verlaufen ausgenommen im Temperaturbereich von 30°C bis 35°C, nahezu parallel, was vermutlich darauf zurückzuführen ist, dass die Anti-RNP-Antikörper ab 35°C eine höhere Bindungsstärke besitzen als die Nachweisfluoreszenzmarkierung Anti-humanes IgG-Atto647N. Nach Verrechnung der relativen Fluoreszenz des Referenzanalyten ergibt sich eine Schmelztemperatur von 44°C für Anti-R060-Antikörper bezogen auf die Werte 3 M, 4 M und 5 M NaCl im Temperaturbereich zwischen 50°C und 60°C. Die Dissoziation ist unter diesen Dissoziationsbedingungen abgeschlossen, weshalb als Messbereich für die Berechnung des Schmelzpunktes eine relative Analytfluoreszenz von 1,2 angenommen wird, zwischen dem graphisch ermittelten maximalen Fluoreszenzwert 1,9 und dem minimalen etwa 0,7.

Der Messbereich der spezifischen Fluoreszenz bei 30°C verringert sich von 0 M NaCl bis 5 M NaCl für die Anti-Ro60-Antikörper des untersuchten Serums. Auffällig ist der sprunghafte Abfall der relativen Fluoreszenzmesswerte des Analyten Anti-Ro60-IgG-Antikörper nach Zugabe von NaCl ab Konzentrationen höher 2 M. Dies spiegelt sich ebenso in der Abnahme der graphisch ermittelten Schmelztemperatur des Analyten Anti-Ro60 im NaCl-Stufengradienten wider: 0 M (44°C), 1 M (39°C), 2 M (36°C), 3 M (34°C), 4 M (32,5°C), 5 M (32°C). Die Fluoreszenz des Referenzanalyten verringerte sich ebenfalls (Daten nicht gezeigt).

### Beispiel 4:

### Nachweis der Bindung eines Lektins an immobilisierte Oligosaccharide und Bestimmung der Bindungsstärke im kombinierten Monosaccharid-Temperaturgradienten

Zur Herstellung der Beadassays werden die biotinylierten Oligosacharide GM1, GM2, GD2, GD3 (Elicityl) an mittels Carbodiimid-Kopplung Avidin-beschichtete Beads, verschiedener Populationen Carboxy-modifizierter, dual-colour-kodierter Beads (PolyAn) gekoppelt. Als Referenzanalyt wird biotinyliertes humanes Serumalbumin (Sigma) in gleicher Weise an eine weitere Beadpopulation gekoppelt. Durch mehrere Waschschritte werden nicht an Beads gebundene Bestandteile der jeweiligen Suspension entfernt. Aliquots jeder Suspension werden gemischt und in polylysinbeschichtete Kavitäten (Diapops-Verfahren, Nunc) von 8er-Modulen (NukleoLink^{®}, ThermoFisher Scientific) gegeben, so dass ca. 50 Beads jeder Population je Kavität nach Durchführung aller Waschschritte der nachfolgenden Testdurchführung vorhanden sind. Nach Sedimentation der Beads wird der Überstand vorsichtig entfernt, um die 8er-Module bei Raumtemperatur zu trocknen und danach bis zur Testdurchführung bei 4°C dunkel zu lagern.

Zur Vorbereitung der Testdurchführung wird der Fluoreszenzfarbstoff Atto647N (**SEQ ID NO. 3**, Atto-Tec) an das Lektin *Helix* pomatia-Agglutinin (Sigma Aldrich) nach Standardverfahren gekoppelt (HPA-Atto647N) und daraus eine Lektinstammlösung hergestellt. Die Stammlösung in PBS-T verdünnt und jeweils 50 µL in eine der vorbereiteten Beadassay-Kavitäten pipettiert und für 1 h inkubiert und anschließend mittels PBS-T gewaschen. Danach erfolgt die ortsaufgelöste Fluoreszenzmessung der Kavitäten im CS300 in den der CA100 integriert wurde bei Raumtemperatur. Die Steuerung des Messgerätes und des Thermocyclers sowie die Aufnahme und Verarbeitung der Bilddaten bis hin zur Ausgabe der relativen Fluoreszenzmesswerte erfolgt entsprechend Herstellerangaben mit Hilfe der Steuer-und Analysesoftware CP1.0. Danach wird jeweils in parallelen Ansätzen 1 µM bis 100 µM N-Acetyl-Galactosamin in PBS-T dazugegeben und die Messung nach 30 min bei Raumtemperatur wiederholt. Danach wird die Temperatur in 0,5°C-Schritten von 35 °C bis 70 °C erhöht. Innerhalb jeder Temperaturstufe erfolgt eine Bildaufnahme sowie Auswertung der Bilddaten. Nach dem Abschluss aller Messschritte wurden die Daten exportiert und in EXCEL graphisch aufbereitet.

Die relativen Fluoreszenzmesswerte verringerten sich mit zunehmender Konzentration des inhibitorisch wirkenden Monosaccharids N-Acetyl-Galactosamin. Durch schrittweise Erhöhung der Temperatur im Reaktionsansatz verringern sich die relativen Fluoreszenzmesswerte weiter ebenso, wie die Schmelztemperaturen des gebundenen Lektins.

### Beispiel 5:

### Nachweis der Bindung von DNA-Doppelstranq Autoantikörpern an Zellstrukturen Crithidia luciliae und Bestimmung der Bindungsstärke im Temperaturgradienten

Zur Herstellung der Beadassays werden humanes Serum Albumin (Sigma) und humanes IgG (Sigma) mittels Carbodiimid-Kopplung an verschiedene Populationen Carboxy-modifizierter, dual-colour-kodierter Beads (PolyAn) gekoppelt. Durch mehrere Waschschritte werden nicht an Beads gebundene Bestandteile der jeweiligen Suspension entfernt.

Crithidien werden in polylysinbeschichtete Kavitäten (Diapops-Verfahren, Nunc) von 8er-Modulen (Nukleolink, ThermoFisher) gegeben und sedimentiert. Der Überstand wird abgenommen und es erfolgt eine Fixierung mit Methanol und anschließend mit Paraformaldehyd. Danach werden die Kavitäten dreimal gewaschen. Aliquots jeder Beadsuspension werden gemischt und in die Crithidien beschichteten Kavitäten gegeben, so dass ca. 50 Beads jeder Population je Kavität nach Durchführung aller Waschschritte der nachfolgenden Testdurchführung vorhanden sind. Nach Sedimentation der Beads wird der Überstand vorsichtig entfernt, um die 8er-Module bei Raumtemperatur zu trocknen und danach bis zur Testdurchführung bei 4°C dunkel zu lagern.

Zur Testdurchführung werden Patientenseren 1:100 in PBS-T, welches 1 % humanes Serumalbumin enthält, verdünnt und jeweils 50 µL in eine der vorbereiteten Beadassay-Kavitäten pipettiert und für 1 h inkubiert. Nach dreimaligem Waschen mittels PBS-T wird Anti-human-lgG-Atto647N-Konjugat (Nachweisfluoreszenzmarkierung) verdünnt in PBS-T in die Kavitäten pipettiert und für 1 h inkubiert. Nach dreimaligem Waschen werden 50 µL PBS-T in die Kavitäten gegeben. Danach erfolgt die ortsaufgelöste Fluoreszenzmessung der Kavitäten im CS300 in den der Thermocycler CA100 integriert wurde bei Raumtemperatur. Die Steuerung des Messgerätes und des Thermocyclers sowie die Aufnahme und Verarbeitung der Bilddaten bis hin zur Ausgabe der relativen Fluoreszenzmesswerte erfolgt entsprechend Herstellerangaben mit Hilfe der Steuer- und Analysesoftware CP1.0. Danach wird die Temperatur in 0,5°C-Schritten von 35 °C bis 70 °C erhöht. Innerhalb jeder Temperaturstufe erfolgt eine Bildaufnahme sowie Auswertung der Bilddaten. Die relativen Fluoreszenzmesswerte der Crithidien wird durch Analyse der aufgenommenen Bilder mit dem Mikrospot-Analyzer (Firma Attomol) vorgenommen. Die ausgegebenen Rohdaten werden anschließend mit EXCEL aufbereitet, um die Anzahl der Chritidien und deren Helligkeit zu bestimmen. Die PK und NK werden analysiert wie in Beispiel 1 beschrieben.

Beim analysierten Patientenserum wurde ein Schmelztemperatur für die Anti-dsDNA-Antikörper von 42°C festgestellt.

### Beispiel 6:

### Einzelantikörpernachweis von Antikörpern gegeben citrullienierte Peptide mittels Nachweisfluoreszenzmarkierung die mit Rolling-Circle-Amplification (RCA) vervielfältigt wurde

Zur Herstellung eines Einzelmolekülnachweises werden das citrullinierte Filaggrin-Peptid-Antigen (**SEQ ID NO. 5**) (Biosynthan) über EDC-Kopplung entsprechend Standardverfahren (DIAPOPS, Nunc) an die feste Phase separater Kavitäten von Nukleolinkplatten gekoppelt.

Zur Immobilisierung des Referenzanalyten wird humanes Serumalbumin (Sigma) bzw. humanes IgG (Sigma) mittels Carbodiimid-Kopplung an verschiedenen Populationen Carboxy-modifizierter, dual-colour-kodierter Beads (PolyAn) gekoppelt. Durch drei Waschschritte werden nicht an Beads gebundene Bestandteile der jeweiligen Suspension entfernt. Aliquots jeder Suspension werden gemischt und in die zuvor mit Peptid beschichteten Kavitäten (Diapops-Verfahren, Nunc) von 8er-Modulen (Nukleolink, ThermoFisher) gegeben, so dass ca. 50 Beads jeder Population je Kavität nach Durchführung aller Waschschritte der nachfolgenden Testdurchführung vorhanden sind. Nach Sedimentation der Beads wird der Überstand vorsichtig entfernt, um die 8er-Module bei Raumtemperatur zu trocknen und danach bis zur Testdurchführung bei 4°C dunkel zu lagern.

Zur Testdurchführung werden Patientenseren 1:100 in PBS-T verdünnt und jeweils 50 µL in eine der der für jedes Peptid vorbereiteten Beadassay-Kavitäten pipettiert und für 1 h inkubiert. Nach dreimaligem Waschen mittels PBS-T wird Anti-human-lgG-Biotin-Konjugat (Nachweisfluoreszenzmarkierung, Seramun) verdünnt in PBS-T in die Kavitäten pipettiert und für 1 h inkubiert. Nach erneutem Waschen erfolgt eine Inkubation mit Avidin verdünnt in PBS-T für 30 min und erneutem Waschen mit PBS-T bevor die Inkubation mit biotinyliertem Fangprimer (**SEQ ID NO. 1**) und RCA-Template (**SEQ ID NO. 2**) verdünnt in PBS-T für 30 min erfolgt. Nach erneutem Waschen in PBS-T wird die Ligationssonde (**SEQ ID NO**. **4)** und T4 Ligase im Ligationspuffer des Herstellers (NEB) 30 min inkubiert und erneut in PBS-T gewaschen. Danach wird Phi29-Polymerase nach Angaben des Herstellers (NEB) im Reaktionspuffer sowie die fluoreszenzmarkierte Nachweissonde in die Kavitäten gegeben und für 4 h bei 37 °C inkubiert.

Nach dreimaligem Waschen werden 50 µL PBS-T in die Kavitäten gegeben. Danach erfolgt die ortsaufgelöste Fluoreszenzmessung der Kavitäten im CS300 (Attomol) bei Raumtemperatur, in den der Thermocycler CA100 integriert wurde. Die Steuerung des Messgerätes und des Thermocyclers sowie die Aufnahme und Verarbeitung der Bilddaten bis hin zur Ausgabe der relativen Fluoreszenzmesswerte erfolgt entsprechend Herstellerangaben mit Hilfe der Steuer- und Analysesoftware CP1.0. Danach wird die Temperatur in 0,5°C-Schritten von 35 °C bis 70 °C erhöht. Innerhalb jeder Temperaturstufe erfolgt eine Bildaufnahme. Nach Abschluss der Messungen werden die Bilder in die Software Microspot-Analyzer (Attomol) übertragen, durch die automatisch die Auswertung der Bilddaten erfolgt. Nach dem Abschluss der Auswertung aller Bilddaten, werden die Daten, , wie die SpotHelligkeit oder der Spotdurchmesser, exportiert und in EXCEL rechnerisch und graphisch aufbereitet. Die Ergebnisse sind in Fig. 4 dargestellt und erläutert. Fig. 4A zeigt die fluoreszierenden Spots aus einem Patientenserum gebundener IgG-Antikörper die an citrullinierte Filaggrin-Peptid-Antigen gebunden haben. Fig. 4B zeigt von der Software Microspot-Analyzer erkannte Antikörper-Bindungsorte von Anti-citrullinierte Filaggrin-Peptid-Antigen-Antikörpern. Durch Erhöhung der Temperatur bis 60°C nimmt die Zahl der Microspots ab, was Folge des Loslösens der Antikörper von an der Unterlage immobilisierten citrulliniertes Filaggrin-Peptid-Antigen-Molekülen ist. lösen sich nach und nach Antikörper von der Unterlage ab. Die Zahl der Spots hat sich bei 60°C auf etwa 55 % des Ausgangswertes reduziert. Der Referenzanalyt humanes IgG bindet im Gegensatz zur Negativkontrolle stark die Nachweisfluoreszenzmarkierung, die sich bei der Dissoziation ähnlich wie in Beispiel 2 verhält. Die Schmelztemperatur für Anti- citrulliniertes Filaggrin-Peptid-Antigen -Antikörper liegt bei 59°C, da sich bei diesen Temperaturen die Zahl der fluoreszierenden Spots halbiert hat.

### Peptidsequenzen für den Einzelmolekülnachweis:

### Oligonukleotidsequenzen für die RCA:

5'-biotinylierter RCA-Fangprimer (**SEQ ID NO. 1**):
   5'- PTTTTTTTTTTTTTTTTTGATACTCTCGTACCTTGAGTG -3'
Lineares RCA-Template (**SEQ ID NO. 2**): 
5’–P–GATCTGCACTGTACTAGTACACTCAAGGTACGAGAGTATCACTGCGTTGCGTA

TATTTCGTTGCGAACGCAGTACTAGTC –3’
Fluoreszenzmarkierte Nachweissonde, Atto647N (**SEQ ID NO. 3**):
   5'- GTTGCGTATATTTCGTTGCG -3'
Ligationssonde (**SEQ ID NO. 4**):
   5'- CTAGTACAGTGCAGATCGACTAGTACTGCG -3'
Citrulliniertes Filaggrin-Peptid-Antigen (**SEQ ID NO. 5**):
   C-term > N-Terminus: Aminohexan -GC*SHQEST-Cit-GRS-Cit-GRSGRC*

## Patentansprüche

1. Verfahren zum Nachweis und zur Bestimmung der Bindungsstärke wenigstens eines Analyten in einer Probe, wobei
A) an einer festen Oberfläche, an räumlich definierten Positionen Komplexe a) und b) immobilisiert werden, umfassend
a) ein oder mehrere Targetmoleküle, an denen der Analyt spezifisch gebunden ist und
b) ein oder mehrere Referenzanalyten;
wobei die Komplexe a) und b) mindestens eine Nachweisfluoreszenzmarkierung aufweisen;
B) eine Messung der Nachweisfluoreszenzmarkierung der Komplexe a) und b) erfolgt;
C) eine Dissoziation der Bindung des Analyten vom Targetmolekül sowie der Bindung der Nachweisfluoreszenzmarkierung vom Referenzanalyten und vom Analyten simultan erfolgt;
D) eine Messung der Nachweisfluoreszenz vor und während der Dissoziation ortsaufgelöst an mindestens zwei verschiedenen Messpunkten erfolgt;
E) die Nachweisfluoreszenzverringerung des mindestens einen Referenzanalyten mit der Nachweisfluoreszenzverringerung des mindestens einen Analyten verrechnet wird und die Bindungsstärke des Analyten als indirekt proportionale Abhängigkeit zur Nachweisfluoreszenzverringerung des mindestens einen Analyten ausgedrückt wird.

2. Verfahren nach Anspruch 1, **dadurch gekennzeichnet, dass** feste Oberflächen planare, transparente Oberflächen eines Trägermaterials sind, ausgewählt aus der Gruppe Objektträger, Biochip, Fluidzelle, Mikrotestplatte oder Einzelkavität einer "break apart"-Mikrotestplatte.

3. Verfahren nach Anspruch 1 oder 2, **dadurch gekennzeichnet, dass** die Komplexe a) und b) getrennt voneinander oder im Gemisch in definierten zweidimensionalen Positionen an der festen Oberfläche des Trägermaterials punkt- oder linienförmig gebunden oder an fluoreszenz- und/oder größenkodierten Mikropartikeln, zufällig räumlich verteilt gebunden werden oder in histologischen Gewebeschnitten oder in Zellen bereits gebunden vorliegen.

4. Verfahren nach einem der vorgehenden Ansprüche, **dadurch gekennzeichnet, dass** Targetmolekül und Referenzanalyt an fluoreszenz- und/oder größenkodierten Beads gebunden und die Beads zufallsverteilt oder geordnet verteilt an die feste Oberfläche des Trägermaterials permanent immobilisiert sind.

5. Verfahren nach einem der vorgehenden Ansprüche, **dadurch gekennzeichnet, dass** mindestens ein Waschschritt der Komplexe a) und b) mit einem Waschpuffer vor der Inkubation mit der Nachweisfluoreszenzmarkierung erfolgt.

6. Verfahren nach einem der vorgehenden Ansprüche, **dadurch gekennzeichnet, dass** die Nachweisfluoreszenzmarkierung einen Analyt-Binder und einen Fluoreszenzfarbstoff umfasst.

7. Verfahren nach einem der vorgehenden Ansprüche, **dadurch gekennzeichnet, dass** die Messung der Nachweisfluoreszenzmarkierung in B) und D) ortsaufgelöst erfolgt.

8. Verfahren nach einem der vorgehenden Ansprüche, **dadurch gekennzeichnet, dass** die Messung der Nachweisfluoreszenzmarkierung in B) mit einem Multiplex-Detektionssystem, ausgewählt aus der Gruppe Biochips, Dot-ELISA, Suspensions-Beadassays oder Festphasen-Beadassays, erfolgt.

9. Verfahren nach einem der vorgehenden Ansprüche, **dadurch gekennzeichnet, dass** die Dissoziation in C) chemisch und/oder physikalisch erfolgt.

10. Verfahren nach einem der vorgehenden Ansprüche, **dadurch gekennzeichnet, dass** die Dissoziation in C) durch Dissoziationspuffer als Stufengradient oder als kontinuierlich ansteigender Gradient und/oder durch Temperaturerhöhung als Temperaturstufengradient erfolgt.

11. Verfahren nach einem der vorgehenden Ansprüche, **dadurch gekennzeichnet, dass** für die Messung der Dissoziation in D) ein Thermocycler verwendet wird.

12. Verfahren nach Anspruch 11, **dadurch gekennzeichnet, dass** der Thermocycler mit einem Fluoreszenzmikroskop kombiniert wird.

13. Verfahren nach einem der vorgehenden Ansprüche, **dadurch gekennzeichnet, dass** in E) die Intensität der Nachweisfluoreszenzmarkierung für jeden Messpunkt als Funktion der Zeit bei isothermaler Reaktionsführung oder als Funktion der Temperatur und/oder der Salzkonzentration bei Anwendung eines Stufengradienten aufgezeichnet wird und der Zeitwert oder der Temperaturwert und/oder Salzkonzentrationswert des Wendepunktes oder der halbmaximalen Fluoreszenzintensität oder eines anderen definierten Punktes der resultierenden Messwertreihen als Maß für die Avidität des Analyten ermittelt wird.

14. Kit zum Nachweis und zur Bestimmung der Bindungsstärke wenigstens eines Analyten in einer Probe enthaltend:
i) wenigstens ein Targetmolekül, an denen der Analyt spezifisch binden kann;
ii) wenigstens einen Referenzanalyten;
iii) eine feste Oberfläche, an welche das Targetmolekül und der Referenzanalyt spezifisch binden kann;
iii) wenigstens eine Nachweisfluoreszenzmarkierung, die wenigstens einen AnalytBinder und wenigstens einen Fluoreszenzfarbstoff umfasst sowie
iv) ein oder mehrere Puffersysteme.

15. Verwendung eines automatisierten Systems zum Nachweis und zur Bestimmung der Bindungsstärke wenigstens eines Analyten in einer Probe mittels des Verfahrens nach einem oder mehreren der Ansprüche 1 bis 13.
